# EUROPEAN PATENT APPLICATION

(11) **EP 3 042 681 A1**
(43) Date of publication of application: **13.07.2016**
(21) Application number: 14840317.3
(22) Date of filing: 01.09.2014
(51) Int. Cl.: A61M 15/00

(54) **INHALER**

(30) Priority: 02.09.2013 JP 2013181707; 02.09.2013 JP 2013181708; 28.02.2014 JP 2014039024; 28.02.2014 JP 2014039484
(71) Applicant: Daiwa Can Company, Tokyo 100-7009 (JP)
(72) Inventor: NAKAMURA, Yasuaki, Tokyo 100-7009 (JP)
(74) Representative: Delorme, Nicolas
(86) International application number: PCT/JP2014/072945
(87) International publication number: WO 2015/030242

(57) **Abstract**

An outer body (5) including a mouth portion (11) through which a drug (100) is inhaled, a cartridge portion (6) in a disc shape rotatably supported inside the outer body (5), arranged at equiangular intervals and spirally, and including a plurality of housing portions (26) that seals and houses the drug (10); and a nozzle portion (7) provided inside the outer body (5), formed so as to be able to open the housing portion (26) by being driven and fluidly connects an inside of the housing portion (26) and the mouth portion (11) by opening the housing portion are included.

## Description

### Technical Field

The present invention relates to an inhaler used to inhale a powder.

### Background Art

Currently, a method of using an inhaler to administer powder drugs to patients with respiratory diseases is known. As such an inhaler, Jpn. Pat. Appln. KOKAI Publication No.2013-509893 discloses a configuration including a cartridge portion having a plurality of housing portions, each encapsulating a dose of drug, an opening means of the housing portion, and a mouth portion as a mouth piece connected to the housing portion.

The inhaler described in Jpn. Pat. Appln. KOKAI Publication No. 2013-509893 is configured to have the housing portions arranged at a predetermined angle and to sequentially open the housing portions by rotating the cartridge portion through operations by an operation member engaged by ratchet teeth.

Since an inhaler is carried by the user and used a plurality of times, an inhaler small in size and having as many housing portions as possible is demanded. Then, an inhaler having housing portions arranged spirally as disclosed by Jpn. PCT National Publication No. 2002-535048 is known. Also, an inhaler having housing portions arranged doubly as disclosed by Jpn. PCT National Publication No. 2002-536080 is known.

Such an inhaler can administer a drug to a patient by one housing portion being opened by the opening means and the drug being inhaled from the mouth portion when used. Also, such an inhaler is configured to be used by the mouth portion being put in the user's mouth and thus, a cover placed over the mouth portion is provided to prevent the mouth portion from being stained while carried.

### Citation List

### Patent Literatures

[Patent Literature 1] Jpn. Pat. Appln. KOKAI Publication No. 2013-509893
[Patent Literature 2] Jpn. PCT National Publication No. 2002-535048
[Patent Literature 3] Jpn. PCT National Publication No. 2002-536080

### Disclosure of Invention

### Technical Problem

In the aforementioned inhalers, a housing portion needs to be moved to a position where the housing portion is opened to open the housing portion. Thus, the mechanism of the inhaler to move a housing portion to a predetermined position for the opening means to open the housing portion becomes complicated. Particularly if the number of housing portions is increased, the mechanism to align the housing portion to be opened becomes more complicated. If the mechanism becomes more complicated, the number of components increases and assembly steps increase, leading to higher manufacturing costs of the inhaler. In addition, the operation of the user becomes complicated.

Thus, an object of the present invention is to provide an inhaler capable of including a plurality of housing portions and allowing the configuration thereof to be simplified.

### Solution to Problem

As an aspect of the present invention, an inhaler includes an outer body including a mouth portion through which a drug is inhaled, a cartridge portion in a disc shape rotatably supported inside the outer body, arranged at equiangular intervals and spirally, and including a plurality of housing portions that seals and houses the drug, and a nozzle portion provided inside the outer body, formed so as to be able to open the housing portion by being driven and fluidly connects an inside of the housing portion and the mouth portion by opening the housing portion.

### Advantageous Effects of Invention

The present invention can provide an inhaler capable of including a plurality of housing portions and allowing the configuration thereof to be simplified.

### Brief Description of Drawings

FIG. 1 is a front view showing the configuration of an inhaler according to a first embodiment of the present invention.
FIG. 2 is a rear view showing the configuration of the inhaler.
FIG. 3 is a rear view showing the configuration of the inhaler.
FIG. 4 is a top view showing the configuration of the inhaler.
FIG. 5 is an exploded perspective view showing the configuration of the inhaler.
FIG. 6 is a perspective view showing the configuration of the inhaler by removing a cover used for the inhaler.
FIG. 7 is a perspective view showing a principal configuration of a body portion used for the inhaler.
FIG. 8 is a perspective view showing the principal configuration of the body portion.
FIG. 9 is a sectional view showing the configuration of principal portions of an operation unit used for the cover and the inhaler used in the body portion.
FIG. 10 is a perspective view showing the configuration of a cartridge portion and a nozzle portion used for the inhaler.
FIG. 11 is a sectional view showing the configuration of the cartridge portion and the nozzle portion.
FIG. 12 is an exploded perspective view showing the configuration of the cartridge portion.
FIG. 13 is a perspective view showing the configuration of the nozzle portion.
FIG. 14 is a sectional view showing the configuration of an outer nozzle and an inner nozzle used for the nozzle portion.
FIG. 15 is a side view showing the configuration of the inner nozzle.
FIG. 16 is a perspective view showing the configuration of a lever member used for the operation unit.
FIG. 17 is a perspective view showing the configuration of a gear member used for the operation unit.
FIG. 18 is an explanatory view showing the configuration of the body portion, the operation unit, and the nozzle portion.
FIG. 19 is an explanatory view showing the configuration of the body portion, the operation unit, and the nozzle portion.
FIG. 20 is an explanatory view showing the configuration of the cartridge portion, the operation unit, and the nozzle portion.
FIG. 21 is an enlarged perspective view showing the principal configuration of the cartridge portion and the gear member.
FIG. 22 is a perspective view showing the configuration the cartridge portion, the operation unit, and an indicator used for the inhaler.
FIG. 23 is a side view showing the configuration the cartridge portion, the operation unit, and the indicator from the side of the indicator.
FIG. 24 is an explanatory view showing the principal configuration used for an inhaler according to a second embodiment of the present invention.

### Mode for Carrying Out the Invention

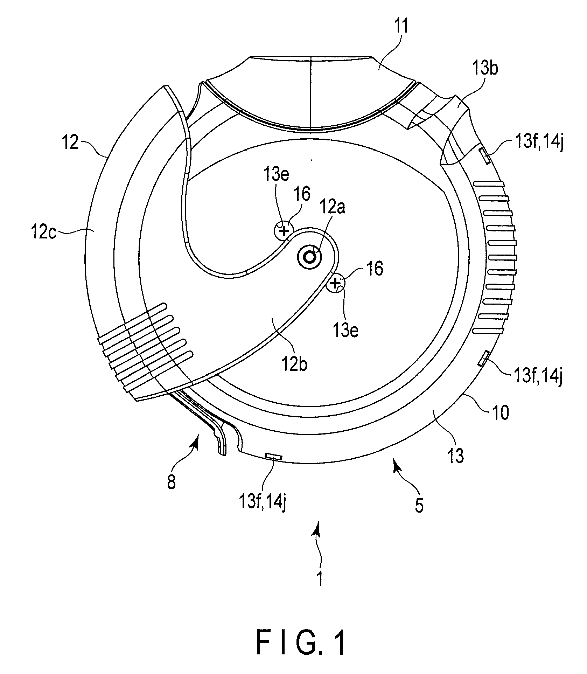

Hereinafter, the configuration of an inhaler 1 according to the first embodiment of the present invention will be described using FIGS. 1 to 23.

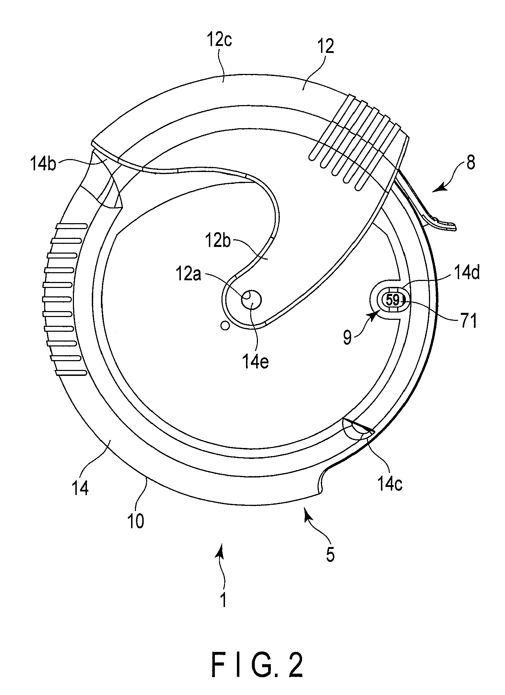

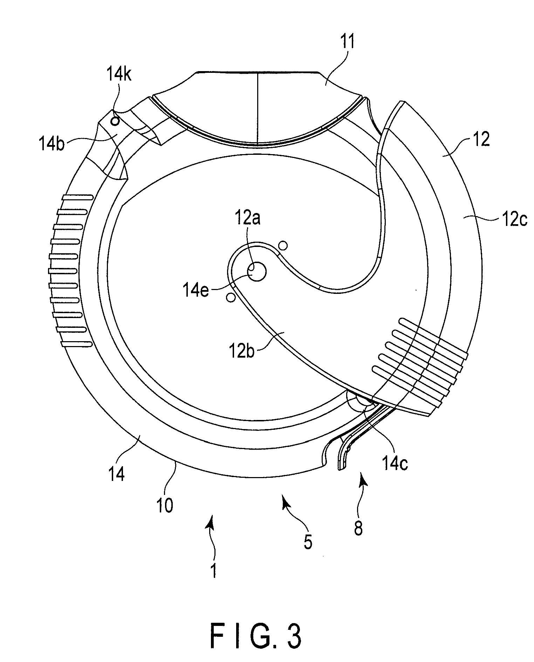

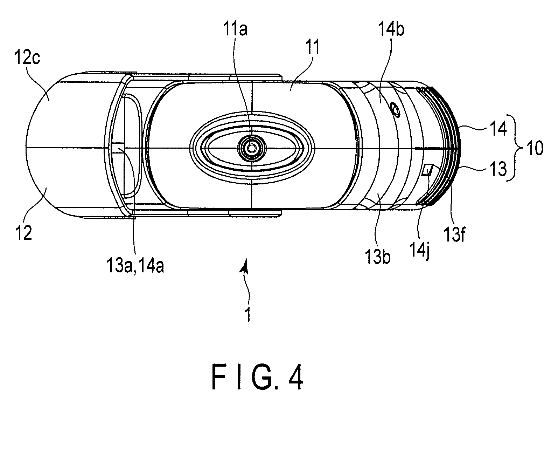

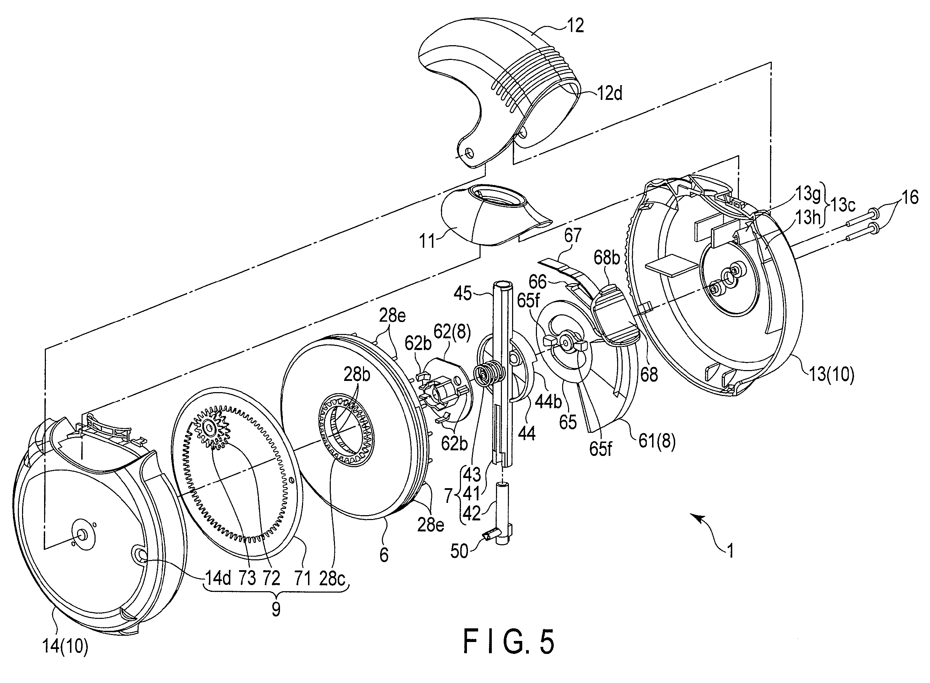

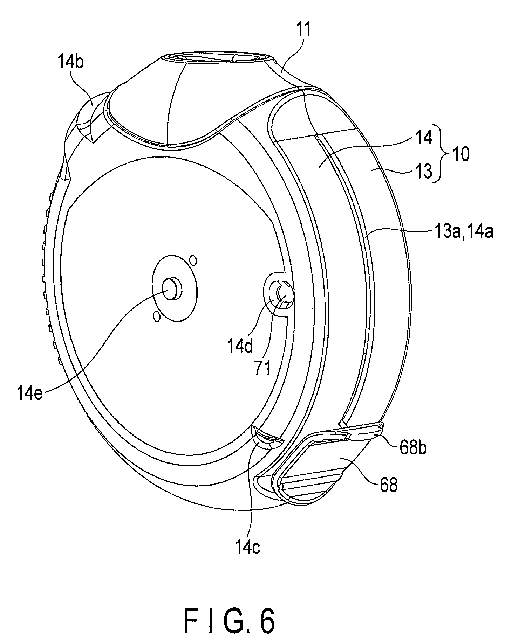

FIG. 1 is a front view of the configuration of the inhaler 1 according to the first embodiment showing a state when used, FIG.2 is a rear view of the configuration of the inhaler 1 showing a standby state, FIG. 3 is a front view of the configuration of the inhaler 1 in a state when used, FIG. 4 is a top view of the configuration of the inhaler 1 in a state when used, FIG. 5 is an exploded perspective view showing the configuration of the inhaler 1, and FIG. 6 is a perspective view showing the configuration of the inhaler 1 by removing a cover 12 used for the inhaler 1.

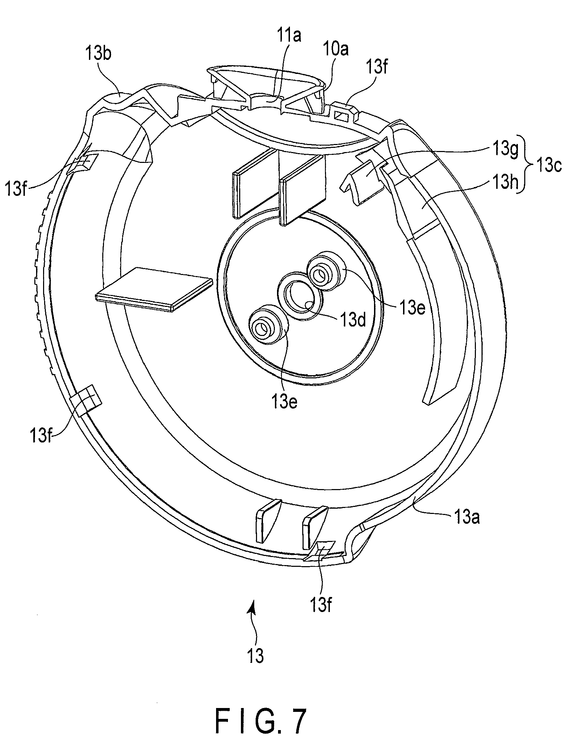

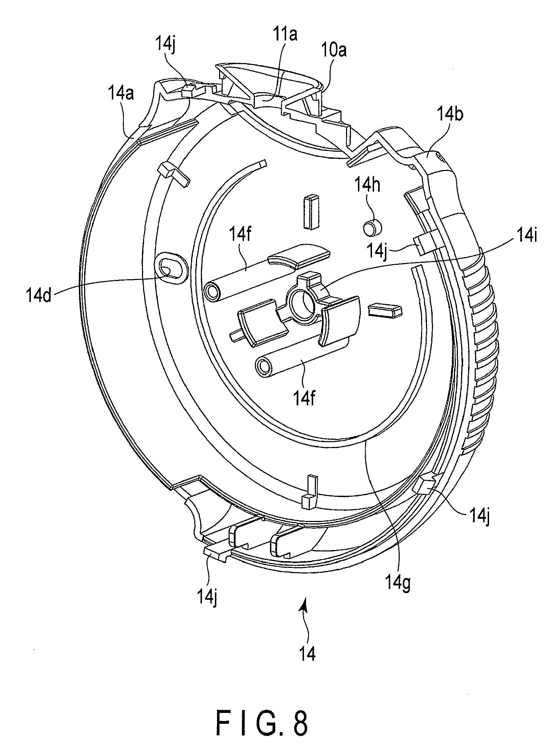

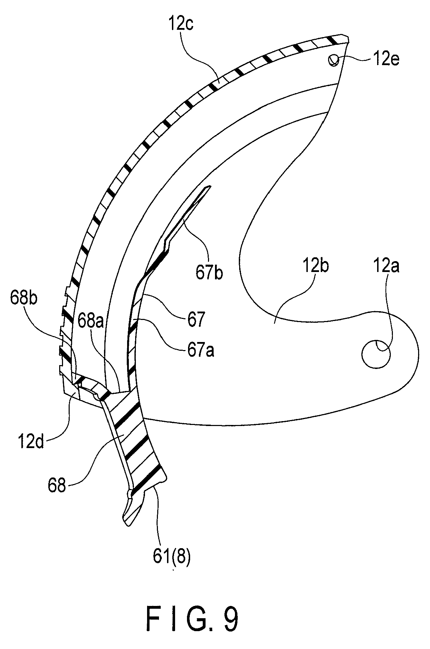

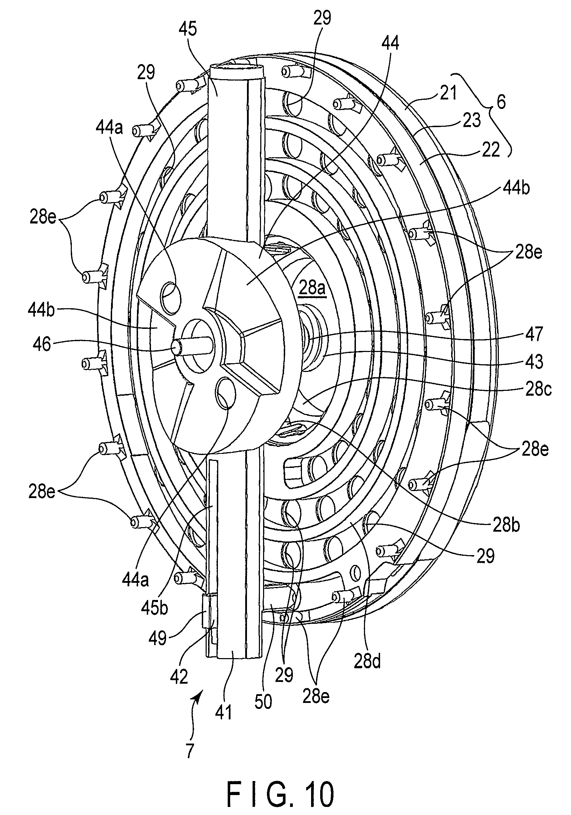

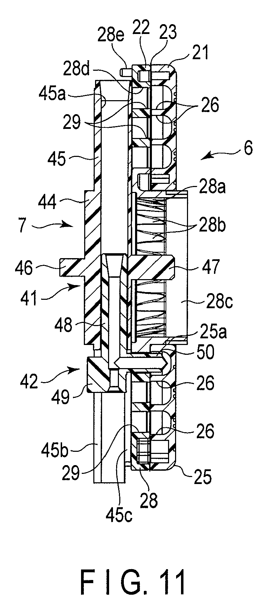

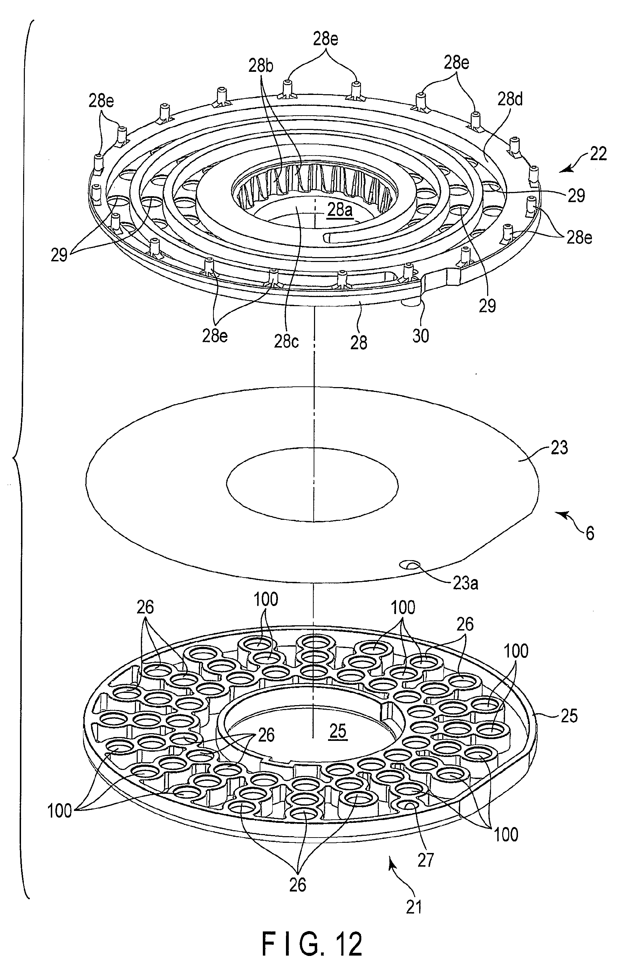

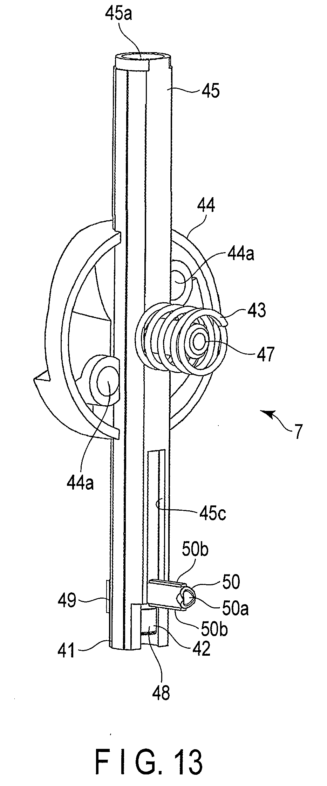

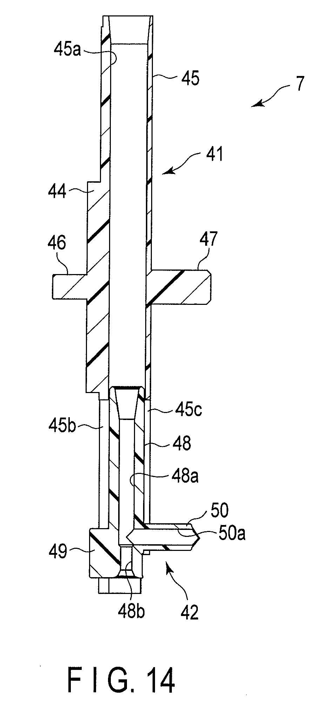

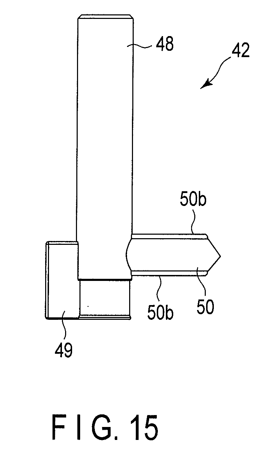

FIG. 7 is a perspective view showing a principal configuration of a body portion 10 used for an outer body 5 of the inhaler 1, more specifically, the configuration of a first body portion 13, FIG. 8 is a perspective view showing the configuration of a second body portion 14 used in the body portion 10, FIG. 9 is a sectional view showing the configuration of the cover 12 of the outer body 5 used for the inhaler 1 and an operation body 68 of an operation unit 8, FIG. 10 is a perspective view showing the configuration of a cartridge portion 6 and a nozzle portion 7 used for the inhaler 1, FIG. 11 is a sectional view showing the configuration of the cartridge portion 6 and the nozzle portion 7, FIG. 12 is an exploded perspective view showing the configuration of the cartridge portion 6, FIG. 13 is a perspective view showing the configuration of the nozzle portion 7, FIG. 14 is a sectional view showing the configuration of an outer nozzle 41 and an inner nozzle 42 used for the nozzle portion 7, and FIG. 15 is a side view showing the configuration of the inner nozzle 42.

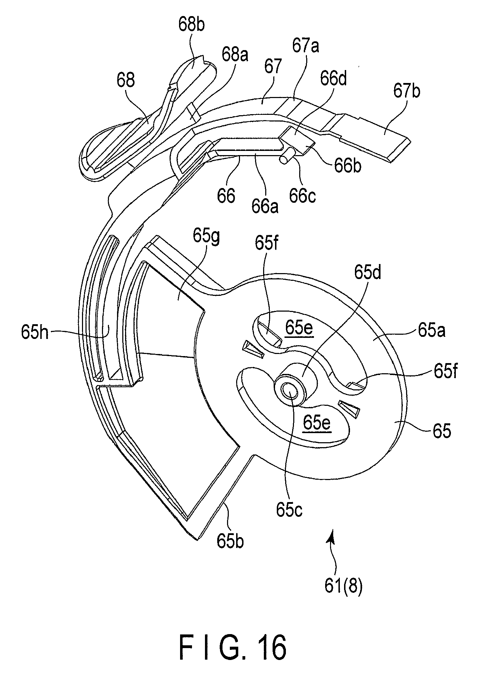

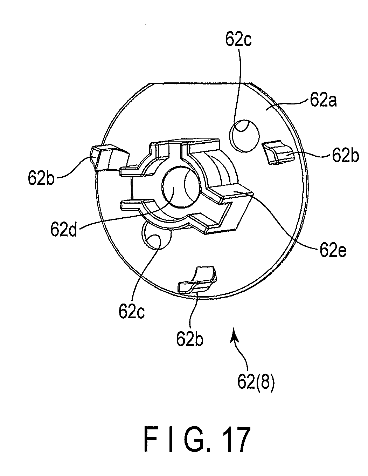

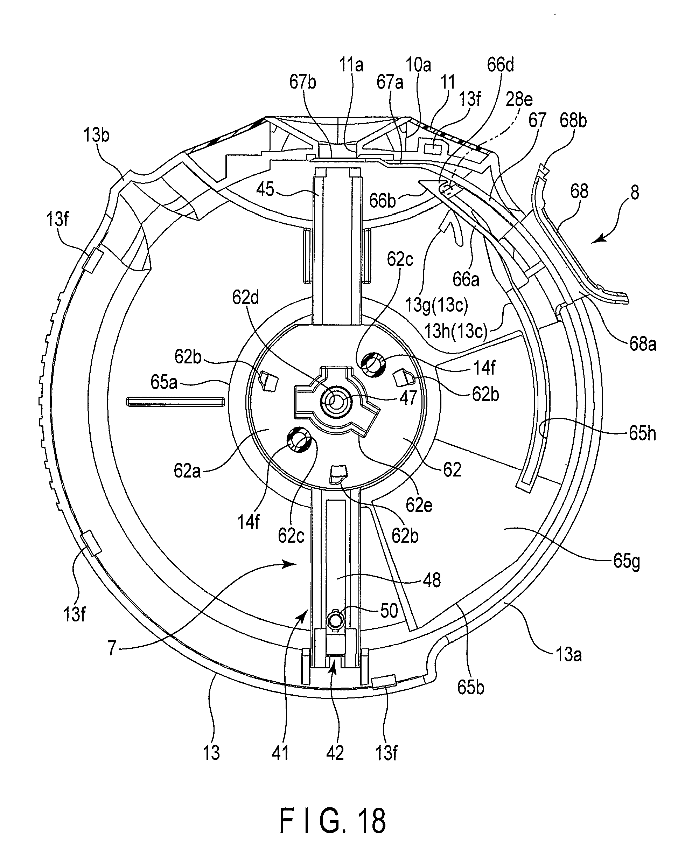

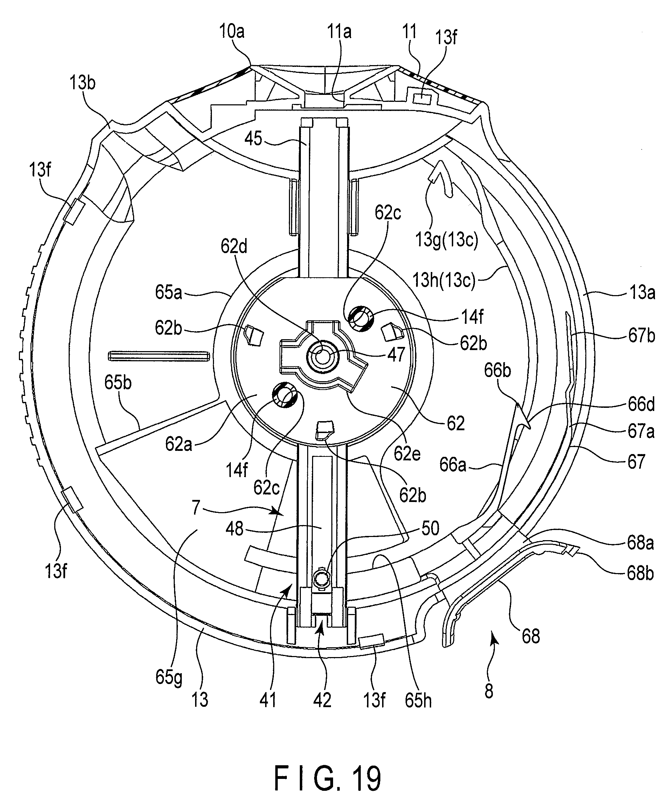

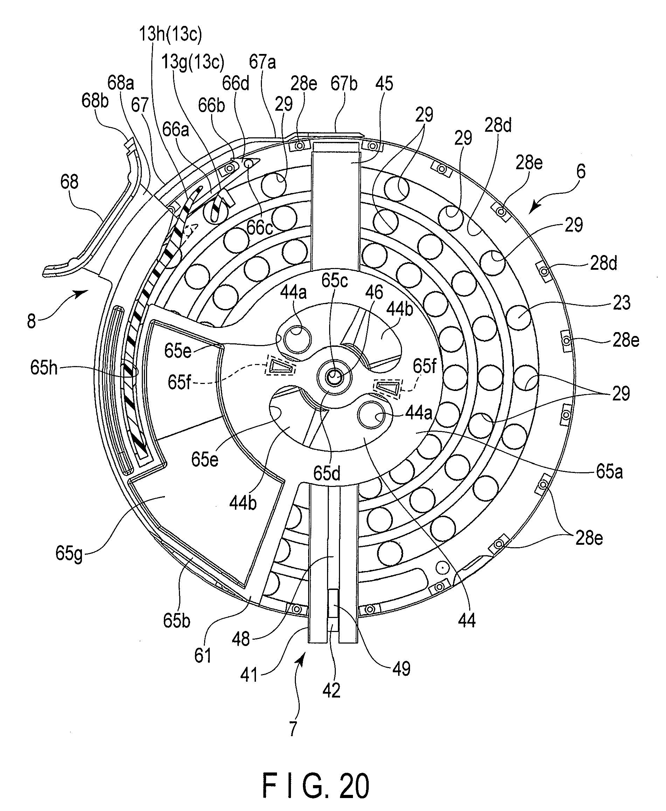

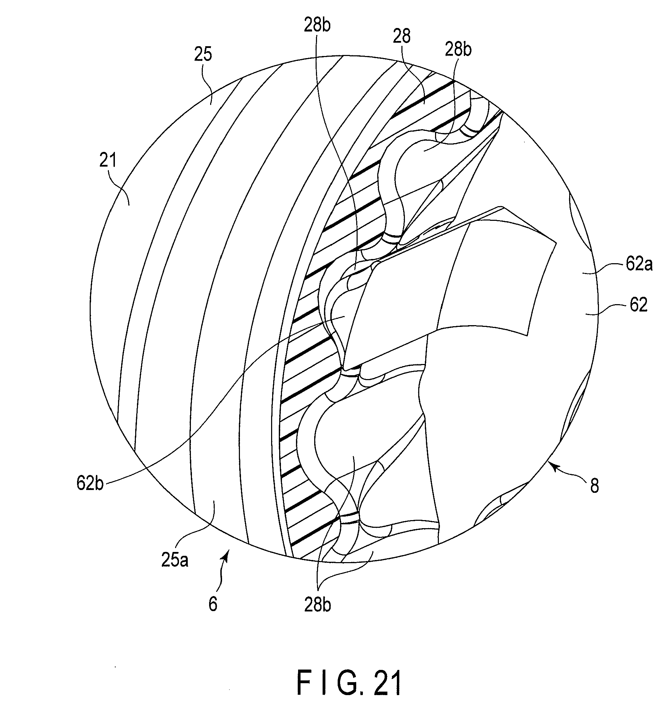

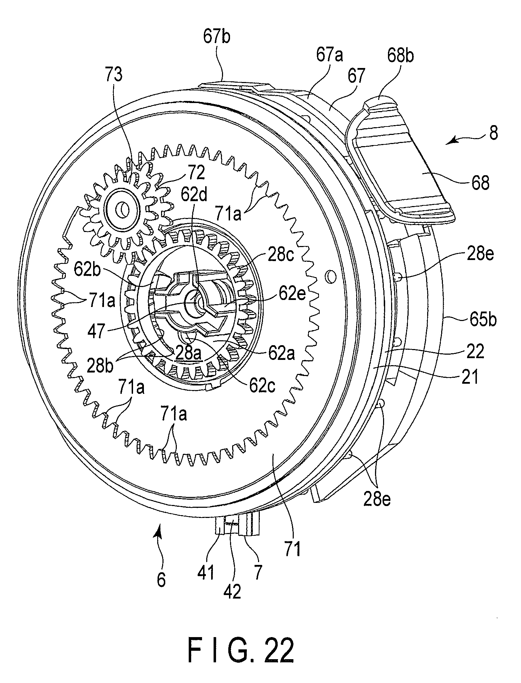

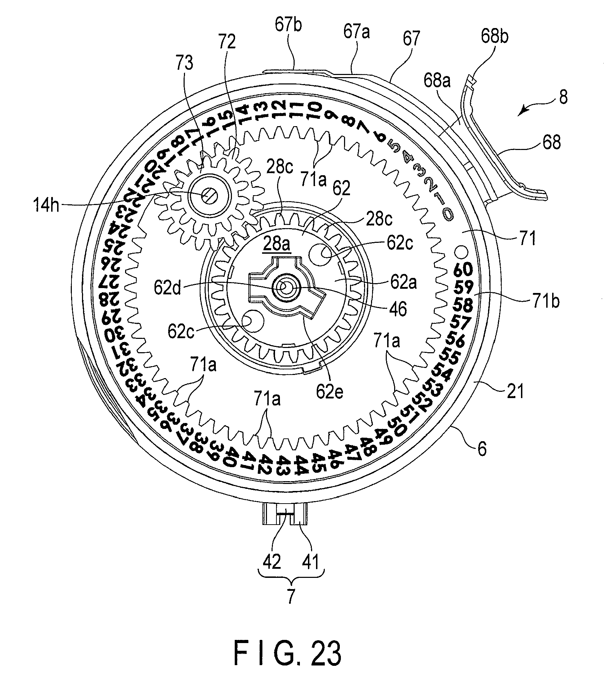

FIG. 16 is a perspective view showing the configuration of a lever member 61 used for the operation unit 8 and FIG. 17 is a perspective view showing the configuration of a gear member 62 used for the operation unit 8. FIG. 18 is an explanatory view of the configuration of the first body portion 13, the nozzle portion 7, and the operation unit 8 showing a standby state, FIG. 19 is an explanatory view of the configuration of the first body portion 13, the nozzle portion 7, and the operation unit 8 showing a state when used, and FIG. 20 is an explanatory view of the configuration of the cartridge portion 6, the nozzle portion 7, and the operation unit 8 showing a standby state. FIG.21 is an enlarged perspective view showing mesh of a gear groove 28b of the cartridge portion 6 and a gear portion 62b of the gear member 62. FIG. 22 is a perspective view showing the configuration of the cartridge portion 6, the operation unit 8, and an indicator 9 used for the inhaler 1 and FIG. 23 is a side view showing the configuration of the cartridge portion 6, the operation unit 8, and the indicator 9 from the side of the indicator 9.

As shown in FIGS. 1 to 6, the inhaler 1 includes the outer body 5, the cartridge portion 6 housed inside the outer body 5, a nozzle portion 7 housed inside the outer body 5, the operation unit 8, and the indicator 9. The inhaler 1 is a container for inhaling a drug 100 of powder. The user inhales the drug 100 of powder stored in a plurality of locations of the cartridge portion 6 in a predetermined quantity via the nozzle portion 7 by operating the operation unit 8 of the inhaler 1.
The outer body 5 covers the body portion 10 accommodating the cartridge portion 6, the nozzle portion 7, and the operation unit 8 and a portion of the nozzle portion 7 and includes a mouth portion 11 in which a hole portion 11a constituting a portion of a channel for the user to inhale a drug and the cover 12 placed over the mouth portion 11 and formed rotatably with respect to the body portion 10.

The body portion 10 is formed in a disc shape. The body portion 10 includes the first body portion 13 and the second body portion 14. As shown in FIGS. 1, 3, and 5, the body portion 10 includes a pair of fastening members 16 supporting a portion of the first body portion 13, the second body portion 14, the nozzle portion 7, and the operation unit 8 on the center side thereof. The first body portion 13 and the second body portion 14 of the body portion 10 are integrally assembled by the fastening members 16.

The first body portion 13 is formed in a disc shape having a curved sidewall formed by the circumference of a principal surface being projected to one side. With a portion of the sidewall of the first body portion 13 being depressed, the first body portion 13 is formed such that the mouth portion 11 is mountable. A portion of the sidewall of the first body portion 13 constitutes a portion of the hole portion 11a of the mouth portion 11. The first body portion 13 includes a first notch portion 13a extended along the circumferential direction of the sidewall on one side thereof and a first protuberance 13b provided in a region adjacent to the mouth portion 11 of the sidewall on the other side to make a gap between an end of the cover 12 and the first body portion 13 narrower.

As shown in FIG. 7, the first body portion 13 includes a guide portion 13c capable of guiding movement of an engaging portion 66 described below of the operation unit 8, an insertion hole 13d into which a fifth shank 65d described below of the operation unit 8 is inserted, a pair of seats 13e to support the fastening members 16 after being inserted from an outer surface side of the first body portion 13, and a plurality of engagement holes 13f engaged with a portion of the second body portion 14. The first body portion 13 also includes a rib or the like that guides the arrangement of the nozzle portion 7 on the inner surface thereof.

The first notch portion 13a is configured to be able to arrange a portion of the operation unit 8 and also guide movement of the operation unit 8. The first protuberance 13b is a protrusion arranged in the first body portion 13 to make a gap between an end of the cover 12 and the first body portion 13 narrower when the cover 12 is positioned to be placed over the mouth portion 11.

As shown in FIGS. 18 to 20, the guide portion 13c is a protrusion having an arc-shaped outer surface and configured to be able to guide the engaging portion 66 along an outer circumference of the cartridge portion 6 and toward the center side of the first body portion 13 when the outer body 5, the cartridge portion 6, the nozzle portion 7, the operation unit 8, and the indicator 9 are integrally assembled.

The guide portion 13c is provided, as shown in FIG. 7, on the inner surface of the first body portion 13, in other words, on a surface opposite to the second body portion 14 of the first body portion 13. The guide portion 13c includes a first guide portion 13g that guides movement of a head portion 66b described below of the engaging portion 66 of the operation unit 8 by engaging with the head portion 66b and a second guide portion 13h that guides movement of the head portion 66b by engaging with a guide protrusion 66c described below of the head portion 66b of the operation unit 8.

The first guide portion 13g guides, as shown in FIGS. 18 and 20, the head portion 66b to a position where the head portion 66b of the operation unit 8 can engage with a pin 28e described below of the cartridge portion 6. The first guide portion 13g guides the head portion 66b along a circumferential direction of the cartridge portion 6 and between the center side of the cartridge portion 6 from the pin 28e and the pin 28e.

The second guide portion 13h guides, as shown in FIGS. 19 and 20, movement of the head portion 66b engaged with the pin 28e on a locus of movement on which the cartridge portion 6 can rotated by engaging with the guide protrusion 66c.

The second guide portion 13h also guides the head portion 66b along the circumferential direction of the cartridge portion 6 and toward the center side of the cartridge portion 6 on a locus on which the pin 28e and the head portion 66b can be disengaged after the head portion 66b moves a predetermined distance in a state in which the pin 28e and the head portion 66b are engaged. The second guide portion 13h also guides the head portion 66b in the circumferential direction on the center side from the pin 28e of the cartridge portion 6 while maintaining a state in which the pin 28e and the head portion 66b are disengaged. The second guide portion 13h also guides the head portion 66b to a position where the head portion 66b abuts on the first guide portion 13g when an operation to return the operation unit 8 to the original position is performed.

The fifth shank 65d described below of the operation unit 8 is inserted into the insertion hole 13d. The seat 13e is formed such that the fastening members 16 can be inserted thereinto and the head of the fastening members 16 can be supported.

The second body portion 14 is formed in a disc shape having a curved sidewall formed by the circumference of a principal surface being projected to one side. With a portion of the sidewall being depressed, the second body portion 14 is formed such that the mouth portion 11 is mountable. A portion of the sidewall of the second body portion 14 constitutes a portion of the hole portion 11a of the mouth portion 11.

The second body portion 14 includes a second notch portion 14a extended along the circumferential direction of the sidewall on one side thereof and opposite to the first notch portion 13a of the first body portion 13 and a second protuberance 14b opposite to the first protuberance 13b to make a gap between the cover 12 and the body portion 10 narrower. The second body portion 14 includes a regulation portion 14c provided at some midpoint of the second notch portion 14a next thereto and a window portion 14d through which the inside of the body portion 10 can visually be identified from outside. The second body portion 14 includes a first shank 14e in a cylindrical shape on an outer surface thereof provided coaxially with the rotation center of the cartridge portion 6 and the operation unit 8 and a locking recess 14k that engages with a locking protrusion 12e described later of the cover 12.

Also, the second body portion 14 includes a protruding portion 14f in a cylindrical shape into which the fastening member 16 can be inserted and a rib 14g in an arc shape rotatably supporting an indicator panel 71 in an annular shape described below of the indicator 9. The second body portion 14 includes a second shank 14h supporting a second gear 72 and a third gear 73 described below of the indicator 9, a rib 14i engaged with the gear member 62 described below of the operation unit 8, and a plurality of claw portions 14j engaged with the plurality of engagement holes 13f of the first body portion 13. The second body portion 14 has the protruding portion 14f, the rib 14g, the second shank 14h, the rib 14i, and the claw portion 14j on a surface opposite to the first body portion 13.

The second notch portion 14a forms a slit opening of a predetermined width by being arranged opposite to the first notch portion 13a. The second notch portion 14a is formed such that a portion of the operation unit 8 can be arranged therein and the movement of the operation unit 8 can be guided.

The second protuberance 14b is a protrusion arranged in the second body portion 14 to make a gap between an end of the cover 12 and the body portion 10 narrower when the cover 12 is positioned to be placed over the mouth portion 11 b being arranged opposite to the first protuberance 13b. The regulation portion 14c is a protrusion arranged on a portion of the outer surface of the second body portion 14 so as to be able to regulate movement of the cover 12 after the cover 12 moves from a position where the mouth portion 11 is covered therewith to a position separated from the mouth portion 11.

The window portion 14d constitutes a portion of the indicator 9 and is opposed to a portion of the indicator panel 71 of the indicator 9 arranged inside the body portion 10. The first shank 14e rotatably supports the cover 12 pivotally. A pair of the protruding portions 14f is provided opposite to the seat 13e and formed, as shown in FIGS. 8, 19, and 20, so as to be insertable into the outer nozzle 41 described below of the nozzle portion 7 and the gear member 62 described below of the operation unit 8.

The rib 14g is a protrusion in an arc shape that supports an inner circumferential portion of the indicator panel 71. The second shank 14h is a cylindrical protrusion rotatably supporting the second and third gears 72, 73 pivotally. The rib 14i has a cylindrical shape and includes a plurality of protrusions on the outer circumferential surface thereof to arrange the gear member 62 in a predetermined position by being engaged with the gear member 62. The claw portion 14j has a return, for example, at a tip thereof. The claw portion 14j is inserted into the engagement hole 13f and the return is engaged with the engagement hole 13f.

The locking recess 14k is formed in the second protuberance 14b. The locking recess 14k is a recess capable of engaging with the locking protrusion 12e of the cover 12.

The first body portion 13 and the second body portion 14 described above are fixed by the fastening members 16 by interposing the cartridge portion 6, the nozzle portion 7, the operation unit 8, and the mouth portion 11 therebetween. The first body portion 13 and the second body portion 14 house a portion of the cartridge portion 6, the nozzle portion 7, and the operation unit 8 therein. The first body portion 13 and the second body portion 14 also constitute a mounting portion 10a on a portion of the outer circumference of which the mouth portion 11 can be mounted and constituting a portion of the hole portion 11a of the mouth portion 11.

The mouth portion 11 is a so-called mouth piece put in the user's mouth. The mouth portion 11 is fixed to the mounting portion 10a of the body portion 10. The mouth portion 11 is integrally formed by, for example, engaging two divided members. The mouth portion 11 internally includes the hole portion 11a. More specifically, the mouth portion 11 has the hole portion 11a formed by the first body portion 13 and the second body portion 14 arranged internally. The mouth portion 11 connects to the nozzle portion 7 housed in the first body portion 13 and the second body portion 14 via the hole portion 11a. The mouth portion 11 is formed, for example, in a shape in which the outer surface on the first body portion 13 side fits to the upper lip of the user. Also, the mouth portion 11 is formed in a shape in which the outer surface on the second body portion 14 side fits to the lower lip of the user.

The cover 12 has a cross section formed in a substantial U shape and is provided in the body portion 10 so as to be rotatable with respect to the mouth portion 11. More specifically, the cover 12 includes a pair of base portions 12b having an opening 12a as the rotation center, a cover portion 12c provided like stretching over the pair of base portions 12b and placed over the mouth portion 11 of the body portion 10, and the locking protrusion 12e formed on the inner surface on one end in the rotation direction of the cover 12.

The pair of base portions 12b is rotatably supported on the body portion 10 pivotally by the first shank 14e of the second body portion 14 inserted into the opening 12a and the fifth shank 65d of the operation unit 8 inserted into the insertion hole 13d of the first body portion 13.

The cover portion 12c has the locking protrusion 12e at one end in the rotation direction. The locking protrusion 12e is clocked onto the locking recess 14k of the second body portion 14. The cover portion 12c formed such that the other end thereof can abut on the regulation portion 14c of the second body portion 14. One end in the rotation direction of the cover portion 12c is placed over the first protuberance 13b and the second protuberance 14b of the body portion 10. Accordingly, the gap between one end of the cover portion 12c and the body portion 10 becomes narrower, which can prevent intrusion of foreign matter into the mouth portion 11. As shown in FIG. 9, the cover portion 12c includes an engagement protrusion 12d capable of engaging with the operation body 68 on the inner surface of the other end, in other words, the end opposite to the operation body 68 described below. The cover portion 12c covers the operation body 68 when only the cover 12 is rotated to recede from the mouth portion 11.

As shown in FIGS. 10 to 12, the cartridge portion 6 includes a drug disc 21, a guide rail 22, and a laminate film 23. The drug disc 21 includes a first base portion 25 having a disc shape and having a circular first opening 25a formed in the center thereof, a plurality of drug pockets 26 provided in the first base portion 25, and a positioning hole 27 provided in the first base portion 25.

The first base portion 25 has the plurality of drug pockets 26 arranged on one principal surface opposite to the guide rail 22.

The drug pocket 26 is formed in a closed-end cylindrical shape. The drug pocket 26 is formed so as to be able to house a predetermined quantity of the drug 100. The predetermined quantity of the drug 100 is a dose administered to the user (patient) at a time. The drug pockets 26 are arranged on one principal surface of the first base portion 25 spirally and radially.

More specifically, the drug pockets 26 are arranged spirally from the center of the first base portion 25 at equiangular intervals, in the present embodiment, at intervals of 18 degrees by being arranged counterclockwise at predetermined intervals with an increasing separation distance from the center side of the first base portion 25 toward the outer circumferential side. A plurality of the drug pockets 26, in the present embodiment, the three drug pockets 26 are arranged in a radial direction from the center of the first base portion 25. Accordingly, the plurality of drug pockets 26 is spirally arranged at equiangular intervals. For example, the lines of the drug pockets 26 are arranged radially at intervals of 18 degrees in 20 lines.

The guide rail 22 includes a second base portion 28 having a disc shape and having a second opening 28a in a circular shape formed in the center thereof, a counter hole 29 provided in the second base portion 28 and opposite to the drug pocket 26 when the guide rail 22 is opposed to the drug disc 21, and a positioning boss 30 provided in a position opposite to the positioning hole 27.

The second base portion 28 includes a plurality of gear groove 28b in a hemiconic shape or a semicircular columnar shape formed on the inner circumferential surface of the second opening 28a and a first gear 28c formed integrally on the other principal surface of the second base portion 28 and formed coaxially next to the second opening 28a.

The gear grooves 28b are provided, like the lines of the drug pockets 26 (a plurality of the pins 28e), on the inner circumferential surface of the second opening 28a at equiangular intervals, in the present embodiment, at intervals of 18 degrees. The gear groove 28b is formed in a shape in which one side face is inclined with respect to the radial direction and the other side face extends along the radial direction. The gear groove 28b meshes with, as shown in FIG. 21, the gear member 62 of the operation unit 8. In FIG. 21, the second base portion 28 is shown by omitting the first gear 28c. The first gear 28c constitutes a portion of the indicator 9.

The second base portion 28 has one principal surface opposite to the drug disc 21 formed flatly. The second base portion 28 includes a guide groove 28d in a spiral shape formed on the other principal surface and passing through all the counter holes 29 continuously and a plurality of pins 28e provided on the outer circumference of the other principal surface.

The guide groove 28d has a width formed equally or a little larger than the inside diameter of the counter hole 29. The drug pocket 26 positioned innermost in the radial direction of the drug disc 21 is positioned at an end on the inner side in the radial direction of the guide groove 28d. The pin 28e is formed in a cylindrical shape and is provided in the same number as the number of lines of the drug pockets 26 and the gear grooves 28b on the outer circumference of the other principal surface of the second base portion 28 at equiangular intervals, in the present embodiment, at intervals of 18 degrees.

The counter hole 29 is provided coaxially with each of the drug pockets 26. The counter hole 29 has an inside diameter formed equally or a little smaller than the inside diameter of the drug pocket 26. The positioning boss 30 is formed in a cylindrical shape and has an outside diameter formed substantially equally to the inside diameter of the positioning hole 27.

The laminate film 23 is formed in an annular shape, more specifically, in the same shape as that of the first base portion 25 and the second base portion 28 and is formed so as to be able to cover the plurality of drug pockets 26. The laminate film 23 is formed as a thin film in which, for example, a PET (polyethylene terephthalate) film, a thin sheet of aluminum, and an adhesive sealant.

The laminate film 23 has a hole portion 23a in a position opposite to the positioning hole 27 of the first base portion 25 and the positioning boss 30 of the second base portion 28. The hole portion 23a has an inside diameter formed equally or a little larger than the inside diameter of the positioning hole 27 and the outside diameter of the positioning boss 30. The laminate film 23 constitutes a housing portion that seals and houses the drug 100 together with the drug pockets 26.

In the cartridge portion 6 as described above, each of the drug pockets 26 of the drug disc 21 is filled with a predetermined quantity of the drug 100 and then the laminate film 23 is welded to an end of the drug pocket 26. Next, the positioning hole 27 and the positioning boss 30 are engaged to fix the guide rail 22 to the drug disc 21. Accordingly, in the cartridge portion 6, the drug disc 21 filled with the drug 100, the guide rail 22, and the laminate film 23 are integrally formed. Also, by sealing the drug pocket 26 with the laminate film 23, the cartridge portion 6 is configured to include a plurality of housing portions of the drug 100 arranged spirally at equiangular intervals.

As shown in FIGS. 10, 11, 13, and 15, the nozzle portion 7 includes the outer nozzle 41, the inner nozzle 42 provided inside the outer nozzle 41, and a coil spring 43 as an energizing member to energize the outer nozzle 41. The nozzle portion 7 forms a fluidly continuous channel of the drug 100 and air from one of the drug pockets 26 to the mouth portion 11.

The outer nozzle 41 includes a fixed portion 44, a first tube portion 45 provided in the fixed portion 44, a third shank 46 provided in the fixed portion 44, and a fourth shank 47 provided in the first tube portion 45 and arranged coaxially with the axial center of the third shank 46.

The fixed portion 44 is formed, for example, in a disc shape and is integrally formed with the first tube portion 45. The fixed portion 44 includes a plurality of hole portions 44a formed in a portion thereof and a pair of first inclined planes 44b provided on one principal surface of the fixed portion 44, more specifically, in a portion of the surface opposite to the first body portion 13 of the outer body 5 The fixed portion 44 has the first tube portion 45 provided integrally on the other principal surface.

A pair of the hole portions 44a is provided in symmetrical positions across the third shank 46 of the fixed portion 44 such that the protruding portion 14f can be inserted thereinto. The hole portion 44a is arranged between the pair of first inclined planes 44b. With the hole portion 44a inserted into the protruding portion 14f, the outer nozzle 41 is regulated in movement in the rotation direction and also is enabled to move along the protruding portion 14f.

The first inclined plane 44b is formed by a portion of the principal surface of the fixed portion 44 being depressed. The first inclined plane 44b is depressed in such a way that when, for example, the front of the first inclined plane 44b is viewed from the first body portion 13 side, the depth gradually increases counterclockwise.

The first tube portion 45 has a channel 45a in a cylindrical shape formed in the center thereof. The first tube portion 45 has one end across the fixed portion 44 arranged opposite to the mouth portion 11 and separated therefrom and a first slit 45b and a second slit 45c arranged opposite to each other formed on the other end across the fixed portion 44.

The first slit 45b is formed by a portion of the first tube portion 45 on one principal surface side of the fixed portion 44 being notched along an axial center direction of the first tube portion 45. The second slit 45c is formed by a portion of the first tube portion 45 on the other principal surface side of the fixed portion 44 being notched along the axial center direction of the first tube portion 45. The second slit 45c is arranged in the first tube portion 45 opposably to the cartridge portion 6. The second slit 45c has a width formed larger than the width of the first slit 45b.

The inner nozzle 42 includes a second tube portion 48 and a plate portion 49 provided in a portion of the outer circumferential surface of the second tube portion 48 and formed on one end side of the second tube portion 48 with the surface direction thereof along the axial center of the second tube portion 48. The inner nozzle 42 includes a third tube portion 50 provided on one end side of the second tube portion 48 in a symmetrical position of the plate portion 49 with respect to the axial center of the second tube portion 48.

The second tube portion 48 has an outside diameter formed equally or a little smaller than the inside diameter of the channel 45a of the first tube portion 45 and is formed so as to be insertable into the first tube portion 45. The second tube portion 48 has a channel 48a formed to connect both ends thereof. The channel 48a has a diameter formed smaller than the diameter of the channel 45a of the first tube portion 45 and the inside diameter on the other end side of the second tube portion 48 is formed larger than the inside diameter from the third tube portion 50 to the opening of the other end side. More specifically, the channel 48a has an inside diameter from the opening on one end side continuing from the channel 45a of the first tube portion 45 to the third tube portion 50 formed larger than the inside diameter from the third tube portion 50 to the opening of the other end side. That is, the second tube portion 48 has at an end open to the body portion 10 a minor diameter channel 48b formed smaller than the inside diameter at an end open to the channel 45a.

The plate portion 49 has a thickness formed equally or a little smaller than the width of the first slit 45b and a portion thereof is formed like a plate projecting outward from the first slit 45b. The plate portion 49 is formed so as to be able to regulate movement in the circumferential direction of the second tube portion 48 by being guided by the first slit 45b when the second tube portion 48 is inserted into the first tube portion 45.

The third tube portion 50 has a channel 50a to connect its end to the channel 48a of the second tube portion 48 formed therein. The channel 50a of the third tube portion 50 has an inside diameter formed as substantially the same diameter of the channel 48a of the second tube portion 48. The third tube portion 50 also has heights 50b formed along the axial center direction on the outer circumferential surface of the third tube portion 50 in a plurality of locations, for example, two locations. The third tube portion 50 is formed continuously from the channel 48a of the second tube portion 48. As shown in FIGS. 11, 14, and 15, the third tube portion 50 is formed by being notched such that the up and down direction of the tip thereof is inclined. The third tube portion 50 is formed such that the sum of the outside diameter thereof and the height of the height 50b is a little smaller than the inside diameter of the drug pocket 26, the inside diameter of the counter hole 29, and the width of the second slit 45c.

The coil spring 43 is formed from metallic materials. One end of the coil spring 43 is supported by the gear member 62 of the operation unit 8 and the other end thereof is supported by the fixed portion 44 of the outer nozzle 41. The coil spring 43 always energizes the outer nozzle 41 in a direction separating from the gear member 62 (second body portion 14).

In the nozzle portion 7 as described above, the inner nozzle 42 is inserted into the outer nozzle 41 and the plate portion 49 and the third tube portion 50 in the inner nozzle 42 are guided to the first slit 45b and the second slit 45c. Accordingly, in the nozzle portion 7, the inner nozzle 42 can move in the axial center direction of the outer nozzle 41 and the rotational movement thereof around the axial center is regulated. In other words, the nozzle portion 7 is formed such that the distance between the end of the outer nozzle 41 and the third tube portion 50 is variable.

The nozzle portion 7 constitutes a Venturi tube using the channels 45a, 48a, 48b, 50a of the first tube portion 45, the second tube portion 48, and the third tube portion 50. With the mouth portion 11 put in the user's mouth and inhaled, the flow of air from the end of the minor diameter channel 48b of the second tube portion 48 and the end of the channel 50a of the third tube portion 50 to the mouth portion 11 via the first tube portion 45 arises in the nozzle portion 7.

The protruding portion 14f inserted into the gear member 62 meshing with the gear groove 28b of the cartridge portion 6 is inserted into the hole portion 44a of the fixed portion 44 such that the gear member 62, the cartridge portion 6, and the coil spring 43 are interposed between the nozzle portion 7 and the second body portion 14. Accordingly, the nozzle portion 7 is opposed to the cartridge portion 6. Also, the nozzle portion 7 is regulated in rotation by the protruding portion 14f and is arranged inside the body portion 10 so as to be movable along the protruding portion 14f. Also, the nozzle portion 7 is always energized by the coil spring 43 in a direction separating from the gear member 62, in other words, in a direction separating from the cartridge portion 6.

As shown in FIGS.16 to 21, the operation unit 8 includes the lever member 61 and the gear member 62. The operation unit 8 is formed such that the cartridge portion 6 and the nozzle portion 7 can be driven by the lever member 61 being rotationally operated by the user.

The lever member 61 includes a base portion 65, an engaging portion 66 provided in the base portion 65, a valve portion 67 provided in the engaging portion 66, and an operation body 68 provided in the valve portion 67. In the lever member 61, the base portion 65, the engaging portion 66, the valve portion 67, and the operation body 68 are integrally formed. The lever member 61 is rotatably formed by the base portion 65 being supported by the third shank 46 of the nozzle portion 7.

The base portion 65 includes a disc portion 65a formed in a disc shape and a projecting portion 65b, which is a portion on the outer circumferential side of the disc portion 65a projecting like a fan. The disc portion 65a includes the fifth shank 65d in a cylindrical shape inserted into the insertion hole 13d of the first body portion 13 and having an insertion hole 65c into which the third shank 46 is inserted formed in the center thereof.

The disc portion 65a includes a pair of openings 65e formed in an arc shape around the fifth shank 65d and a protruding portion 65f formed in a portion of the principal surface opposite to the fixed portion 44, in contact with the first inclined plane 44b of the fixed portion 44, and pressing the first inclined plane 44b. The openings 65e extend in the disc portion 65a along the rotation direction of the operation unit 8 such that the pair of protruding portions 14f can be inserted thereinto. The opening 65e is a clearance to prevent interference between the protruding portions 14f and the base portion 65 when the lever member 61 is operated. The protruding portion 65f is formed such that the height projecting from the disc portion 65a is equal to the moving distance of the nozzle portion 7 with respect to the cartridge portion 6. The protruding portion 65f is a pressing means that moves the nozzle portion 7 in a direction approaching the cartridge portion 6 by pressing the first inclined plane 44b.

The projecting portion 65b includes a second inclined plane 65g provided on the principal surface thereof and a clearance portion 65h in an arc shape formed in a portion of the principal surface. The second inclined plane 65g is formed by projecting from the projecting portion 65b to the side of the nozzle portion 7 and is formed, for example, at the same angle of inclination as that of the first inclined plane 44b.

The second inclined plane 65g is formed such that the height projecting from the base portion 65 is equal to the moving distance of the nozzle portion 7 with respect to the cartridge portion 6 or more and is also formed so as to be able to interfere with the plate portion 49 of the inner nozzle 42 of the nozzle portion 7. The clearance portion 65h is a clearance that allows the projecting portion 65b to be arranged in a non-contact manner around the second guide portion 13h when positioned in the second guide portion 13h provided in the first body portion 13.

The engaging portion 66 includes an extending portion 66a extending from between the base portion 65 and the operation body 68 in a tangential direction of the outer circumference of the base portion 65 (projecting portion 65b), a head portion 66b provided at an end of the extending portion 66a, and a guide protrusion 66c provided in the head portion 66b and engaged with the side face of the second guide portion 13h.

The extending portion 66a is formed so as to be bendable when the head portion 66b or the guide protrusion 66c is guided to the first guide portion 13g or the second guide portion 13h. The extending portion 66a is formed so as to be able to support the head portion 66b in substantially the same position as the pin 28e of the cartridge portion 6 in the radial direction.

The head portion 66b includes a return portion 66d. The return portion 66d is formed engagably with the pin 28e. By engaging with the second guide portion 13h, the movement direction of the head portion 66b is guided. The guide protrusion 66c is formed in a cylindrical shape and provided on the side face opposite to the first body portion 13 of the head portion 66b.

In the engaging portion 66 as described above, when the lever member 61 is rotated by exceeding an angle half the angle between the pins 28e after the head portion 66b is engaged with the pin 28e, the guide protrusion 66c is guided by the second guide portion 13h in the circumferential direction of the cartridge portion 6. Accordingly, the extending portion 66a of the engaging portion 66 is bent inward such that the head portion 66b moves to the center side in the radial direction of the cartridge portion 6. Accordingly, the engaging portion 66 is configured such that head portion 66b separates from the pin 28e and the head portion 66b and the pin 28e are disengaged.

In other words, in the engaging portion 66 in the present embodiment, when the operation body 68 exceeds 9 degrees after the head portion 66b is engaged with the pin 28e, the head portion 66b rotates in the circumferential direction around the axial center of the cartridge portion 6 and also moves in the radial direction toward the axial center of the cartridge portion 6. Accordingly, the head portion 66b moves from the position of the pin 28e to the center side of the cartridge portion 6 to disengage the head portion 66b and the pin 28e.

The valve portion 67 includes an extending portion 67a provided between the engaging portion 66 and the operation body 68 and a valve body 67b provided at the tip of the extending portion 67a. The valve portion 67 is provided on the outer side of the engaging portion 66 in the radial direction. The valve body 67b is formed like a flexible plate. The valve body 67b can shut off the flow (channel) of the drug 100 from the first tube portion 45 to the hole portion 11a by being interposed between an opening end of the first tube portion 45 of the outer nozzle 41 and the hole portion 11a of the mouth portion 11.

The valve portion 67 as described above is a shut-off valve that shuts off the channel between the first tube portion 45 of the nozzle portion 7 and the hole portion 11a of the mouth portion 11 by the valve body 67b being arranged between the opening end of the first tube portion 45 and the hole portion 11a of the mouth portion 11 at all times, in other words, in a standby state when the inhaler 1 is not used. When the operation body 68 is operated, the valve portion 67 recedes from between the opening end of the first tube portion 45 and the hole portion 11a to open the channel between the first tube portion 45 and the hole portion 11a of the mouth portion 11.

The operation body 68 is formed by projecting from the valve portion 67 in the radial direction. More specifically, the operation body 68 includes a base body 68a arranged in the notch portions 13a, 14a of the body portion 10, movable along the notch portions 13a, 14a, and connected to the valve portion 67. The operation body 68 is formed in a plate shape in which both ends in the circumferential direction of the body portion 10 project outward such that a user's finger can be caught. In addition, the end on the mouth portion 11 side of the operation body 68 is covered with the cover 12. The operation body 68 includes an engaged portion 68b capable of engaging with the engagement protrusion 12d formed at an end of the cover 12 when the operation body 68 is operated or the cover 12 is rotated toward the mouth portion 11. The engaged portion 68b of the operation body 68 is provided in a position on the mouth portion 11 side of the engagement protrusion 12d of the cover 12 when the operation unit 8 is provided in the body portion 10.

As shown in FIG. 17, the gear member 62 includes a base portion 62a formed in a disc shape and a plurality of gear portions 62b in a hemiconic shape or a semicircular columnar shape provided on the outer circumferential side of one principal surface of the base portion 62a and having the same shape as the gear groove 28b.

The gear member 62 also includes a pair of hole portions 62c into which the pair of protruding portions 14f can be inserted and an insertion hole 62d capable of pivotally supporting the fourth shank 47 on the center side of the base portion 62a. The gear member 62 includes a rib 62e engaged with the outer surface of the rib 14i of the second body portion 14 and a spring seat 62f provided on the other principal surface of the base portion 62a shown in FIG. 5 to support the coil spring 43 described below of the nozzle portion 7.

For example, the three gear portions 62b are provided at irregular intervals. The gear portion 62b is formed so as to be able to mesh with the gear groove 28b and also to be able to climb over the gear groove 28b by rotation of the cartridge portion 6 by being bent. The gear portion 62b is formed, like the gear groove 28b, in a shape in which one side face is inclined with respect to the radial direction and the other side face extends along the radial direction. The gear portion 62b can climb over the gear groove 28b only in one direction, more specifically, in the rotation direction of the cartridge portion 6.

The gear member 62 is arranged between the second body portion 14 and the cartridge portion 6 by being inserted into the pair of protruding portions 14f by the hole portion 62c and the movement thereof in the rotation direction is regulated by the fastening member 16 being inserted into the hole portion 62c.

The gear member 62 is provided in the protruding portion 14f such that the gear portion 62b is arranged in a predetermined position by the rib 62e being engaged with the rib 14i of the second body portion 14. In addition, the gear member 62 rotatably supports the cartridge portion 6 by, as shown in FIG. 21, the gear portion 62b being meshed with the gear groove 28b of the guide rail 22.

The nozzle portion 7 and the operation unit 8 constitute an opening means that opens a housing portion (the laminate film 23 and the drug pocket 26) in which the drug 100 is housed of the cartridge portion 6 by a user's operation of the operation unit 8.

As shown in FIGS. 2, 22, and 23, the indicator 9 includes the window portion 14d provided in the second body portion 14, the first gear 28c provided in the second base portion 28 of the cartridge portion 6, and the indicator panel 71 formed in an annular shape and having a plurality of teeth 71a formed on the inner circumference thereof. The indicator 9 includes the second gear 72 meshing with the first gear 28c and the third gear 73 formed integrally with the second gear 72 and meshing with the teeth 71a of the indicator panel 71.

The indicator panel 71 includes an information indicator 71b indicating information, more specifically, 0 to the number equal to the number of the drug pockets 26 of the cartridge portion 6, in the present embodiment, 0 to 60 on the surface opposite to the second body portion 14. The information indicator 71b is provided in the indicator panel 71 by, for example, printing, a seal or the like.

When the cartridge portion 6 is rotated by the operation of the operation unit 8 to be ready for use of the drug 100 in the drug pocket 26, the indicator 9 rotates the indicator panel 71 by a predetermined angle. More specifically, when the cartridge portion 6 is rotated, the rotation is transferred to the indicator panel 71 from the first gear 28c via the second gear 72 and the third gear 73 as transfer members that transfer the rotation of the cartridge portion 6 to rotate the indicator panel 71 by a predetermined angle. The indicator 9 indicates a portion of information of the information indicator 71b, more specifically, one number from the window portion 14d. Accordingly, the indicator 9 is formed so as to be able to indicate the drug pockets 26 that remain (that are not opened).

That is, the indicator 9 is a counter formed so as to be able to indicate the remaining number of times of use of the inhaler 1. If the remaining number of times of use or the number of times of use of the inhaler 1 is known, the indicator 9 may be configured to count down the remaining number of housing portions of the drug 100 (remaining number of times of use) or count up the number of times of use.

Next, the method of using the inhaler 1 configured as described above and inhaling the drug 100 will be described.

First, the user supports the body portion 10 in the left hand with the second body portion 14 side closer to the user and turns the body portion 10 so that the first body portion 13 is directed upward. Next, the user operates the operation body 68 by the thumb of the right hand to move (rotate) the operation body 68 along the notch portions 13a, 14a in a direction separating from the mouth portion 11. At this point, as shown in FIG. 9, the engaged portion 68b of the operation body 68 is engaged with the engagement protrusion 12d of the cover 12 and thus, with the rotation of the operation body 68, the cover 12 rotates in a direction separating from the mouth portion 11 to expose the mouth portion 11.

When the operation body 68 is moved, the lever member 61 rotates around the fifth shank 65d. When the lever member 61 rotates, as shown in FIGS. 18 and 20, the head portion 66b engages with the pin 28e and the engaging portion 66 rotates the cartridge portion 6 in an operation direction of the operation body 68. If, in this state, the operation body 68 is further moved, as shown in FIGS. 19 and 20, the guide protrusion 66c of the head portion 66b moves to the rotation center side along the second guide portion 13h. Accordingly, the head portion 66b moves to the rotation center side and the head portion 66b and the pin 28e are thereby disengaged.

If the cartridge portion 6 rotates by exceeding a predetermined angle, in the present embodiment, 9 degrees while the head portion 66b and the pin 28e are engaged, the gear groove 28b and the gear portion 62b of the gear member 62 are disengaged. Accordingly, the gear portion 62b climbs over the gear groove 28b and the gear portion 62b moves to the gear groove 28b on the secondary side.

Incidentally, the head portion 66b remains engaged with the pin 28e until the gear portion 62b travels a distance to climb over the gear groove 28b. Thus, even if the head portion 66b and the pin 28e are disengaged by the guidance of the second guide portion 13h, the next gear groove 28b and the gear portion 62b are engaged. Accordingly, the cartridge portion 6 rotates by an angle between the gear grooves 28b, in the present embodiment, 18 degrees.

With the rotation of 18 degrees of the cartridge portion 6, the inner nozzle 42 of the nozzle portion 7 is guided along the guide groove 28d to move slightly in the radial direction (axial direction of the first tube portion 45) of the cartridge portion 6. Also with the rotation of 18 degrees of the cartridge portion 6, the drug pocket 26 on the secondary side of the drug pocket 26 having been opposed to the third tube portion 50 is now opposed to the third tube portion 50. Also with the movement of the protruding portion 65f with respect to the first inclined plane 44b due to the rotation of the operation body 68, the protruding portion 65f presses against the first inclined plane 44b.

As a result, the nozzle portion 7 is pressed toward the cartridge portion 6, the coil spring 43 is elastically deformed in a compression direction, and the nozzle portion 7 is moved in a direction approaching the cartridge portion 6 before the laminate film 23 closing the drug pocket 26 being broken by the tip of the third tube portion 50. Accordingly, the third tube portion 50 is inserted into the drug pocket 26 and the inside of the drug pocket 26 fluidly continues to the hole portion 11a of the mouth portion 11 via the channels 45a, 48a, 50a of the first tube portion 45, the second tube portion 48, and the third tube portion 50.

With the movement of the second inclined plane 65g with respect to the nozzle portion 7 due to the rotation of the operation body 68, the second inclined plane 65g interferes with the plate portion 49 to press against the plate portion 49. Accordingly, the plate portion 49 can be pressed by, in addition to pressing of the first inclined plane 44b by the protruding portion 65f, the second inclined plane 65g so that the tip of the third tube portion 50 can be inserted into the drug pockets 26 more reliably.

The user inhales a small quantity of air from the minor diameter channel 48b of the second tube portion 48 and the drug 100 and air from the channel 50a of the third tube portion 50 by breathing in with the mouth portion 11 put in the user's mouth and as a result, the drug 100 is sucked in. At this point, the drug 100 reaches the predetermined site of the user, namely the set arrival setting site of the drug 100, for example, the bronchial tube or pulmonary alveoli set depending on the opening area of the minor diameter channel 48b.

With the rotation of the cartridge portion 6, the indicator panel 71 is rotated by the first gear 28c, the second gear 72, and the third gear 73. Accordingly, the information indicator 71b rotates relative to the window portion 14d and the next number of the number of the information indicator 71b opposite to the window portion 14d is positioned. Accordingly, the current remaining number of the drug pockets 26 of the inhaler 1 can visually be identified.

After inhaling the drug 100, the user operates the cover 12 to move the cover 12 to a position where the cover 12 is placed over the mouth portion 11. While the cover 12 is moved, as shown in FIG. 9, the engagement protrusion 12d of the cover 12 and the engaged portion 68b of the operation body 68 are engaged and thus, the operation body 68 also moves accompanying the movement of the cover 12. With the movement of the operation body 68, the head portion 66b moves along the second guide portion 13h and when the cover 12 moves near the mouth portion 11, the cover 12 moves along the first guide portion 13g. Thus, with the movement of the head portion 66b by being guided by the first guide portion 13g and the second guide portion 13h, as shown in FIGS. 18 and 20, the head portion 66b is arranged in a position allowing the head portion 66b to engage with the pin 28e.

Also, with the movement of the cover 12 to a position where the cover 12 is placed over the mouth portion 11, pressing of the first inclined plane 44b by the protruding portion 65f is released and also pressing of the plate portion 49 by the second inclined plane 65g is released.

As a result, the nozzle portion 7 is moved in a direction separating from the cartridge portion 6 by a restoring force of the coil spring 43. Accordingly, the third tube portion 50 separates from the drug pocket 26 and the counter hole 29 to be arranged in the guide groove 28d. Accordingly, when the operation body 68 is operated in the next use of the inhaler 1, the cartridge portion 6 is made movable by the engaging portion 66 and also the nozzle portion 7 is made movable to connect to the inside of the next drug pocket 26.

By repeating the above operation of the operation body 68, the user can open the housing portion (the drug pocket 26 and the laminate film 23) of the drug 100 to inhale the drug 100 as many times as the number of the drug pockets 26 (number of times of using the inhaler 1).

According to the inhaler 1 configured as described above, in a standby state in which the inhaler 1 is not used, the cover 12 is placed over the mouth portion 11 and also the channel between the nozzle portion 7 and the mouth portion 11 is shut off by the valve body 67b and thus, foreign matter can be prevented from intruding into the nozzle portion 7 from the mouth portion 11. Even if the user erroneously attempts to inhale only by rotating the cover 12 without operating the operation body 68, the hole portion 11a of the mouth portion 11 is closed by deformation of the valve body 67b having flexibility in accordance with the user's suction force. Accordingly, the flow of air is blocked and the user feels resistance so that the user can recognize the erroneous use.

Also, by operating the operation body 68, the inhaler 1 can allow the cartridge portion 6 to rotate by a predetermined angle each time and further can convert motion in the rotation direction of the operation unit 8 into reciprocating motion of the nozzle portion 7 with respect to the cartridge portion 6.

By spirally arranging the drug pockets 26 at intervals of the same angle as the angle of rotation by one operation of the operation body 68 of the cartridge portion 6 and providing the guide groove 28d in a spiral shape, the inner nozzle 42 of the nozzle portion 7 can be moved in the radial direction of the cartridge portion 6 due to the rotation of the cartridge portion 6.

By operating the operation body 68, the inhaler 1 can recede the valve body 67b from between the opening end of the outer nozzle 41 and the hole portion 11a of the mouth portion 11 to connect the nozzle portion 7 and the mouth portion 11. With the movement of the nozzle portion 7 in a direction approaching the cartridge portion 6, the inhaler 1 can also break the laminate film 23 closing the drug pocket 26 by the tip of the third tube portion 50 of the inner nozzle 42 of the nozzle portion 7. Accordingly, the housing portion is opened by the nozzle portion 7 and the inhaler 1 can connect the drug pocket 26 and the mouth portion 11.

The engaged portion 68b of the operation body 68 is engaged with the engagement protrusion 12d of the cover 12 by operating the operation body 68 and thus, the inhaler 1 can expose the mouth portion 11 by rotating the cover 12 in a direction away from the mouth portion 11 without operating the cover 12.

Therefore, the inhaler 1 can perform three operations of exposing the mouth portion 11, opening the channel between the nozzle portion 7 and the mouth portion 11, and connecting the drug pocket 26 and the mouth portion 11 only by operating the operation body 68 so that the user can inhale the drug 100 by simple operations.

Since the inhaler 1 engages with the engagement protrusion 12d of the cover 12 and the engaged portion 68b of the operation body 68, by operating the cover 12 after the use to move the cover 12 to a position where the cover 12 is placed over the mouth portion 11, the operation body 68 is operated by the cover 12. Accordingly, by operating only the cover 12 without operating the operation body 68, the operation body 68 can be moved to a standby state ready for the next use so that the inhaler 1 can be put into a standby state by a simple operation.

Even if the cover 12 is operated in a standby state, the engagement protrusion 12d is not engaged with the engaged portion 68b of the operation body 68 and thus, even if the cover 12 is erroneously operated when the inhaler 1 is carried in a bag or the user holds the inhaler, only the cover 12 is rotated. Therefore, even if an erroneous operation of the cover 12 occurs, the housing portion can be prevented from being opened because the cartridge portion 6 can prevent the rotation.

Thus, the inhaler 1 can prevent unintended opening of the drug pocket 26 (housing portion) caused by rotation of the cartridge portion 6 due to an occurrence of an erroneous operation of the cover 12. As a result, the inhaler 1 can prevent the decrease of the number of times of using the inhaler 1 and leakage of the drug 100 from the mouth portion 11.

Further, opening of the housing portion by an erroneous operation can be prevented and also the drug pockets 26 are provided in separate bodies and the nozzle portion 7 is connected to each of the drug pockets 26 and therefore, the inhaler 1 is configured to allow the user to inhale a predetermined quantity of the drug 100.

With a portion of the tip of the third tube portion 50 notched, the area that comes into contact with the laminate film 23 is decreased, which can make it easier to break the laminate film 23, and also an opening area is increased and therefore, the drug 100 can reliably be moved when inhaled.

The third tube portion 50 is configured by notching a region positioned in an up and down direction in a vertical posture of the inhaler 1, that is, while the mouth portion 11 is arranged upward. Thus, when the inhaler 1 is used, the movement of the drug 100 stored on the lower side in the gravity direction is not blocked and also an opening area can be secured. Further, by providing the height 50b on the outer circumference of the third tube portion 50, the nozzle portion 7 can secure a predetermined gap between the outer circumferential surface of the third tube portion 50 and the inner circumferential surface of the drug pocket 26 and the counter hole 29 so that the flow of air into the drug pocket 26 can be secured. From the above, the drug 100 in the drug pocket 26 can reliably be inhaled by forming the flow of air when inhaled and after inhaling, the drug 100 can be prevented from remaining in the drug pocket 26.

Thus, even if the quantity of the drug 100 encapsulated in the drug pocket 26 is a predetermined quantity of a dose, the user can reliably inhale the predetermined quantity of the drug 100. As a result, there is no need to encapsulate more than the predetermined quantity of the drug 100 in the drug pocket 26 in consideration of the drug 100 that is not inhaled (remaining in the drug pocket 26). Therefore, the manufacturing cost of the inhaler 1 can be reduced.

By making the inner nozzle 42 movable relative to the outer nozzle 41, the nozzle portion 7 makes the channels 45a, 48a, 50a of the nozzle portion 7 extendable and the expansion and contraction of the nozzle portion 7 can be implemented by a simple configuration in which the guide groove 28d of the cartridge portion 6 is caused to guide the inner nozzle 42. Similarly, the housing portion can easily be opened by the nozzle portion 7 by moving the nozzle portion 7 depending on the abutting position of the first inclined plane 44b and the protruding portion 65f

The third tube portion 50 of the inner nozzle 42 of the nozzle portion 7 is arranged inside the guide groove 28d and guided along the guide groove 28d when the operation body 68 is rotated. Thus, when an attempt is made to further rotate the operation body 68 after all the drugs 100 provided along the guide groove 28d are used up, the third tube portion 50 abuts on the end portion of the guide groove 28d to prevent the cartridge portion 6 from rotating. Accordingly, the operation body 68 is also prevented from rotating and thus, the user can clearly recognize that the drugs 100 have been used up.

Opposing the nozzle portion 7 to each housing portion and opening the housing portion can be implemented by a simple operation of the rotation operation of the lever member 61 of the operation unit 8. Therefore, the inhaler 1 can reduce the number of components and the manufacturing cost and also improve usability of the user.

By arranging the drug pockets 26 at equiangular intervals on the first base portion 25, the inhaler 1 can arrange many housing portions by saving space. Since the nozzle portion 7 can efficiently move the drug 100 depending on the shape of the third tube portion 50, the shape of the drug pocket 26 only needs to be able to house a predetermined quantity of the drug 100. Thus, the inhaler 1 can also be reduced in size.

Further, by providing the window portion 14d capable of moving relative to a pair of rail portions 25b provided in a spiral shape as the indicator 9 in the outer body 5, the inhaler 1 can switch the indication of the information indicator 71b accompanying the rotation of the cartridge portion 6. Accordingly, the inhaler 1 can indicate information about the remaining number of the drug pockets 26 in a simple configuration.

The inhaler 1 is configured to use the coil spring 43 formed from a metallic material as an energizing means to energize the outer nozzle 41 to separate the third tube portion 50 from the cartridge portion 6. Therefore, the modulus of elasticity can be prevented from changing after repeated use of the inhaler 1 and the cost thereof can be reduced.

The inhaler 1 rotates the lever member 61 while the pin 28e of the cartridge portion 6 and the head portion 66b of the engaging portion 66 are engaged and also moves the head portion 66b to the center side of the cartridge portion 6 in the radial direction while rotating the head portion 66b by the guide portion 13c after the gear portion 62b climbs over the gear groove 28b. Accordingly, the cartridge portion 6 having rotated by a predetermined angle can be rotated by the predetermined angle by the mesh of the gear groove 28b and the gear portion 62b. In other words, regardless of the rotation angle of the lever member 61, the inhaler 1 can rotate the cartridge portion 6 by the predetermined angle.

By configuring the operation unit 8 to recede the head portion 66b toward the center side of the body portion 10 when the head portion 66b is disengaged from the pin 28e, there is no need to provide a space on the outer circumferential side of the body portion 10 to allow the head portion 66b to recede from the pin 28e. Thus, the inhaler 1 can be prevented from growing in size.

According to the inhaler 1 in the first embodiment of the present invention, as described above, the cartridge portion 6 is moved in the circumferential direction by engaging the engaging portion 66 and the pin 28e and operating the operation body 68. Also, the same operation enables the gear groove 28b of the cartridge portion 6 and the gear portion 62b of the gear member 62 to mesh and the gear portion 62b to climb over the gear groove 28b. In the inhaler 1, after the lever member 61 is rotated by a predetermined angle, the engaging portion 66 is disengaged from the pin 28e by the guide portion 13c and then, the cartridge portion 6 is rotated by a predetermined angle by the mesh of the gear groove 28b and the gear portion 62b. Accordingly, the cartridge portion of the inhaler 1 is rotatable with high precision.

Also according to the inhaler 1, the cover 12 placed over the mouth portion 11 is included and, the cover 12 is movable by operating the operation body 68 for the use of the inhaler 1 and the operation body 68 is movable by operating the cover 12 after the inhaler 1 is used. Accordingly, even if the cover 12 placed over the mouth portion 11 is provided, the inhaler 1 can reduce steps operated by the user while the inhaler 1 is used and after the inhaler 1 is used. Since the engagement protrusion 12d of the cover 12 and the engaged portion 68b of the operation body 68 are engaged in the inhaler 1 only during operation of the operation body 68 when the inhaler 1 is used and during operation of the cover 12 after the inhaler 1 is used, even if the cover 12 is erroneously operated, the cartridge portion 6 can be prevented from rotating. As a result, even if the cover 12 is erroneously operated, the housing portion can be prevented from being opened.

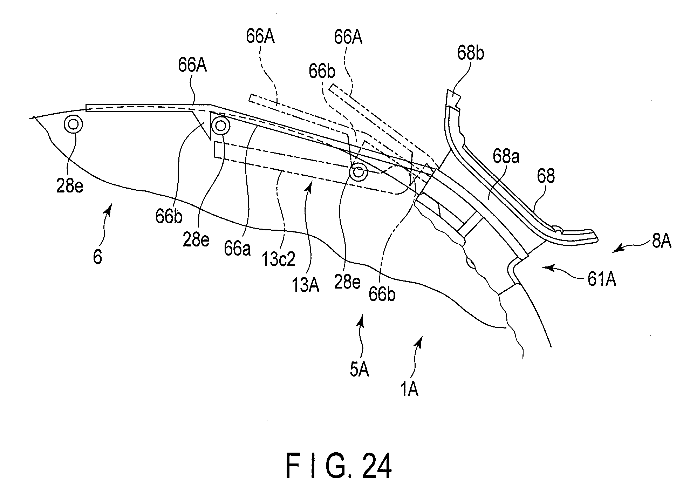

Next, an inhaler 1A according to the second embodiment of the present invention will be described using FIG. 24.

FIG.24 is an explanatory view showing the configuration of a guide portion 13c2 and a lever member 61A used for the inhaler 1A according to the second embodiment of the present invention. In the inhaler 1A according to the second embodiment, the same reference signs are attached to the same components as those of the inhaler 1 according to the first embodiment described above and a detailed description thereof is omitted.

The inhaler 1A includes an outer body 5A, a cartridge portion 6 housed inside the outer body 5A, a nozzle portion 7 housed inside the outer body 5A, an operation unit 8A, and an indicator 9. The inhaler 1A is a container for inhaling a drug 100 of powder. The user inhales the drug 100 of powder stored in a plurality of locations of the cartridge portion 6 in a predetermined quantity via the nozzle portion 7 by operating the operation unit 8A of the inhaler 1A.

The outer body 5A covers a body portion 10A accommodating the cartridge portion 6, the nozzle portion 7, and the operation unit 8 and a portion of the nozzle portion 7 and includes a mouth portion 11 in which a hole portion 11a constituting a portion of a channel for the user to inhale a drug and a cover 12 placed over the mouth portion 11 and formed rotatably with respect to the body portion 10A.

The body portion 10A is formed in a disc shape. The body portion 10A includes a first body portion 13A and a second body portion 14. The body portion 10A includes a pair of fastening members 16 supporting a portion of the first body portion 13A, the second body portion 14, the nozzle portion 7, and the operation unit 8 on the center side thereof. In the body portion 10A, the first body portion 13A and the second body portion 14 are integrally assembled by the fastening members 16.

The first body portion 13A is formed in a disc shape having a curved sidewall formed by the circumference of a principal surface being projected to one side. With a portion of the sidewall being depressed, the first body portion 13A is formed such that the mouth portion 11 is mountable. A portion of the sidewall of the first body portion 13A constitutes a portion of the hole portion 11a of the mouth portion 11. The first body portion 13A includes a first notch portion 13a and a first protuberance 13b.

The first body portion 13A includes a guide portion 13c2 capable of guiding movement of an engaging portion 66A of the operation unit 8A, an insertion hole 13d, a pair of seats 13e, and a plurality of engagement holes 13f. The first body portion 13A also includes a rib or the like that guides the arrangement of the nozzle portion 7 on the inner surface thereof.

As shown in FIG. 24, the guide portion 13c2 is a protrusion formed along the outer circumference of the cartridge portion 6 such that the engaging portion 66A can be guided along the radial direction of the cartridge portion 6 when the outer body 5A, the cartridge portion 6, the nozzle portion 7, the operation unit 8, and the indicator 9 are integrally assembled.

The guide portion 13c2 is provided on the inner surface of the first body portion 13A. The guide portion 13c2 is arranged such that an end thereof is on the outer side of a pin 28e in the radial direction of the cartridge portion 6. The guide portion 13c2 has, for example, an end on the side of an operation body 68 of a lever member 61A described below of the operation unit 8A formed in a substantially cylindrical shape having a curved outer surface and is formed like a plate toward the other end. The guide portion 13c2 is formed by being inclined with respect to a tangent of the outer circumference of the cartridge portion 6 such that the surface direction of a plate region moves from the center side of the cartridge portion 6 to the outer side toward the end having a curved outer surface.

The guide portion 13c2 guides the movement of the head portion 66b by being engaged with one of an extending portion 66a described below of the engaging portion 66A of the operation unit 8A and the head portion 66b. Accordingly, the guide portion 13c2 guides the extending portion 66a or the head portion 66b depending on operation conditions of the operation unit 8A to move the head portion 66b to a position where the head portion 66b is engaged with the pin 28e and a position of disengagement.

As shown in FIG. 24, the operation unit 8A includes the lever member 61A and a gear member 62. The operation unit 8A is formed so as to be able to drive the cartridge portion 6 and the nozzle portion 7 when the user performs a rotation operation of the lever member 61A.

The lever member 61A includes a base portion 65, the engaging portion 66A provided in the base portion 65, and the operation body 68 provided in the engaging portion 66A. In the lever member 61A, the base portion 65, the engaging portion 66A, and the operation body 68 are integrally formed. The lever member 61A is formed rotatably with respect to the third shank 46 by the base portion 65 being supported by the third shank 46 of the nozzle portion 7.

The engaging portion 66A includes the extending portion 66a extending from between the base portion 65 and the operation body 68 in a tangential direction of the outer circumference of the base portion 65 and the head portion 66b provided at a midpoint of the extending portion 66a

The extending portion 66a is formed so as to be bendable to the outer and inner sides in the radial direction of the cartridge portion 6 using the base or therearound extending from between the base portion 65 and the operation body 68 as an axis. The extending portion 66a is formed so as to be able to abut on an end of the guide portion 13c2. By abutting on the end of the guide portion 13c2, the motion of the extending portion 66a bending to the inner side in the radial direction of the cartridge portion 6 is regulated.

The head portion 66b is a return projecting to the side of the operation body 68, in other words, to the center side of the cartridge portion 6. The head portion 66b is formed so as to be able to engage with the pin 28e of the cartridge portion 6 when the lever member 61 moves in the operation direction. The head portion 66b extends, for example, in a direction in which one surface thereof in the rotation direction of the lever member 61A is perpendicular to the tangential direction of the outer circumferential surface of the cartridge portion 6. Also, for example, the head portion 66b extends with the other surface being inclined with respect to the tangential direction of the outer circumferential surface of the cartridge portion 6.

The head portion 66b is formed so as to be able to engage with the pin 28e. After the operation body 68A is rotated by exceeding an angle half the angle between the pins 28e and less than the sum of the angle between the pins 28e and half the angle between the pins 28e on the secondary side after the head portion 66b is engaged with the pin 28e, the engaging portion 66A abuts on an end of the guide portion 13c2 positioned on the outer side of the cartridge portion 6. Accordingly, the extending portion 66a is bent to the outer side in the radial direction of the cartridge portion 6 and correspondingly the head portion 66b is separated from the pin 28e to disengage the head portion 66b and the pin 28e. In the present embodiment, for example, when, after the head portion 66b is engaged with the pin 28e, the operation body 68 rotates by exceeding 9 degrees in the engaging portion 66A, the head portion 66b and the pin 28e are disengaged.

Next, the method of using the inhaler 1A configured as described above will briefly be described.

First, in a standby state in which the inhaler 1A is not used, like the engaging portion 66A indicated by a solid line in FIG. 24, for example, the head portion 66b is engaged with the pin 28e or in a position immediately before being engaged with the pin 28e. When the operation body 68 is moved by a user's operation, the head portion 66b is moved while being engaged with the pin 28e. At this point, like the engaging portion 66A indicated by an alternate long and two short dashes line in FIG. 24, the head portion 66b moves together with the pin 28e while moving to the outer side of the cartridge portion 6 along a plane portion of the guide portion 13c2. When the operation body 68 further moves and rotates by exceeding a predetermined angle, in the present embodiment, 9 degrees, the head portion 66b is disengaged from the pin 28e by the end of the guide portion 13c2. Accordingly, the gear groove 28b and a gear portion 62b of the gear member 62 are disengaged, the gear portion 62b climbs over the gear groove 28b, and the gear portion 62b moves to the gear groove 28b on the secondary side.

With the movement of the gear portion 62b, the cartridge portion 6 is rotated by 18 degrees and the inner nozzle 42 is guided along a guide groove 28d to move slightly in the radial direction (axial direction of the first tube portion 45) of the cartridge portion 6. Also with the rotation of 18 degrees of the cartridge portion 6, a drug pocket 26 on the secondary side of the drug pocket 26 having been opposed to a third tube portion 50 is now opposed to the third tube portion 50. Also with the movement of a protruding portion 65f with respect to a first inclined plane 44b due to the rotation of the operation body 68, the protruding portion 65f presses against the first inclined plane 44b.

As a result, the nozzle portion 7 is pressed toward the cartridge portion 6, a coil spring 43 is elastically deformed in a compression direction, and the nozzle portion 7 is moved in a direction approaching the cartridge portion 6 before a laminate film 23 closing the opposed drug pocket 26 being broken by the tip of the third tube portion 50. Accordingly, the third tube portion 50 is inserted into the drug pocket 26 and the inside of the drug pocket 26 fluidly continues to the hole portion 11a of the mouth portion 11 via channels 45a, 48a, 50a of the first tube portion 45, the second tube portion 48, and the third tube portion 50.

The user inhales the drug 100 by breathing in with the mouth portion 11 put in the user's mouth.

With the rotation of the cartridge portion 6, an indicator panel 71 is rotated by a first gear 28c, a second gear 72, and a third gear 73. Accordingly, the next number of the number of an information indicator 71b opposite to a window portion 14d is now opposed to the window portion 14d.

After the drug 100 is inhaled, the operation body 68 moves accompanying the movement of the cover 12 by the cover 12 being operated by the user. With the movement of the operation body 68, the head portion 66b moves along the guide portion 13c2 and the head portion 66b is arranged in a position where the head portion 66b can be engaged with the next pin 28e.

Also, pressing of the first inclined plane 44b by the protruding portion 65f is released and also pressing of a plate portion 49 by a second inclined plane 65g is released.

As a result, the nozzle portion 7 is separated from the cartridge portion 6 by a restoring force of the coil spring 43. Accordingly, the third tube portion 50 separates from the drug pocket 26 and the counter hole 29 to be arranged in the guide groove 28d. Accordingly, a standby state in which the inhaler 1A can be used next time is entered.

According to the inhaler 1A configured as described above, like the inhaler 1 according to the first embodiment described above, the cartridge portion 6 is moved in the circumferential direction by engaging the engaging portion 66A and the pin 28e and operating the operation body 68. Also, the same operation enables the gear groove 28b of the cartridge portion 6 and the gear portion 62b of the gear member 62 to mesh and the gear portion 62b to climb over the gear groove 28b. In the inhaler 1A, after the lever member 61A is rotated by a predetermined angle, the engaging portion 66A is disengaged from the pin 28e by the guide portion 13c and then, the cartridge portion 6 is rotated by a predetermined angle by the mesh of the gear groove 28b and the gear portion 62b. Accordingly, the cartridge portion of the inhaler 1A is rotatable with high precision.

Also according to the inhaler 1A, the cover 12 placed over the mouth portion 11 is included and, the cover 12 is movable by operating the operation body 68A when the inhaler 1A is used and the operation body 68 is movable by operating the cover 12 after the inhaler 1A is used. Accordingly, even if the cover 12 placed over the mouth portion 11 is provided, the inhaler 1A can reduce steps operated by the user while the inhaler 1A is used and after the inhaler 1A is used. An engagement protrusion 12d of the cover 12 and an engaged portion 68b of the operation body 68 are engaged in the inhaler 1A only during operation of the operation body 68 when the inhaler 1A is used and during operation of the cover 12 after the inhaler 1A is used. Thus, even if the cover 12 is operated erroneously, the housing portion can be prevented from being opened by preventing the rotation of the cartridge portion 6.

However, the present invention is not limited to the above embodiments. In the examples described above, the configuration in which the drug 100 is inhaled by the inhaler 1 being put in the user's mouth are described, but the present invention is not limited to such examples. For example, the mouth portion 11 may be formed in a shape enabling the user to inhale from the nostril.

In the above examples, the configuration in which the inhaler 1 forms the nozzle portion 7 as a Venturi tube having the minor diameter channel 48b, but the present invention is not limited to such examples. Depending on the arrival position of the drug 100 when inhaled from the inhaler 1, a configuration that closes the channel without providing the minor diameter channel 48b may be adopted or the minor diameter channel 48b may be configured to have the same diameter as the channel 48a.

Further in the above examples, the configuration of the third tube portion 50 in which a portion of the tip thereof is notched is described, but the tip of the third tube portion 50 may be formed in a tube-like shape without being notched. However, the third tube portion 50 preferably has a portion of the tip notched because the opening area of the third tube portion 50 opposed to the drug 100 is increased by notching a region positioned below more when the inhaler 1 is used. In addition, various modifications can be made without deviating from the spirit of the present invention.

## Claims

1. An inhaler comprising:
an outer body including a mouth portion through which a drug is inhaled;
a cartridge portion in a disc shape rotatably supported inside the outer body, arranged at equiangular intervals and spirally, and including a plurality of housing portions that seals and houses the drug; and
a nozzle portion provided inside the outer body, formed so as to be able to open the housing portion by being driven and fluidly connects an inside of the housing portion and the mouth portion by opening the housing portion.

2. The inhaler according to claim 1, wherein
the nozzle portion includes an outer nozzle formed movably with respect to the cartridge portion and
an inner nozzle movable inside the outer nozzle along an axial center of the outer nozzle.

3. The inhaler according to claim 2, wherein
the housing portion includes a drug pocket that houses the drug and a laminate film that seals the drug pocket and
the inner nozzle is formed so as to be able to abut on and break the laminate film with movement of the outer nozzle with respect to the cartridge portion.

4. The inhaler according to claim 3, wherein the cartridge portion includes a guide rail that passes through the plurality of housing portions and in which a tip of the inner nozzle is arranged, further comprising:
an operation unit that rotates the cartridge portion by an angle between the housing portions and also moves the outer nozzle with respect to the cartridge portion.

5. The inhaler according to claim 4, wherein
the operation unit moves the outer nozzle in a direction approaching the cartridge portion and
the outer nozzle includes between the outer nozzle and the cartridge portion an elastic portion that energizes between the outer nozzle and the cartridge portion.

6. The inhaler according to claim 5, wherein
the outer nozzle includes a slit along the axial center thereof at one end and
the inner nozzle is formed in a cylindrical shape and includes a plate portion provided in a portion of an outer circumferential surface thereof and guided along the slit when the inner nozzle moves inside the outer nozzle and a tube portion provided in a portion of the outer circumferential surface thereof and forming a channel in a direction perpendicular to the axial center of the inner nozzle.

7. The inhaler according to claim 6, wherein the inner nozzle has an inside diameter at the end of the channel along the axial center thereof formed smaller than the inside diameter of the tube portion.

8. The inhaler according to claim 7, wherein the tube portion is formed by a portion of the tip thereof being notched.

9. The inhaler according to claim 1, further comprising: an operation unit capable of rotating the cartridge portion by an angle between the plurality of housing portions and which moves the nozzle portion with respect to the cartridge portion.

10. The inhaler according to claim 9, wherein
the nozzle portion includes an outer nozzle formed movably with respect to the cartridge portion, an inner nozzle movable inside the outer nozzle along an axial center of the outer nozzle, a first inclined plane provided in the outer nozzle, and an elastic portion provided between the outer nozzle and the cartridge portion to energize between the outer nozzle and the cartridge portion and
the operation unit includes a base portion formed rotatably, an operation body provided in the base portion, a protruding portion provided in the base portion, in contact with the first inclined plane, and pressing the first inclined plane, and a second inclined plane formed at a same angle as the first inclined plane, capable of abutting on the inner nozzle, and movable to the inner nozzle by rotation of the base portion.

11. The inhaler according to claim 10, wherein
the cartridge portion includes a pin provided on an outer circumference thereof and arranged at intervals of the same angle as the angle between the housing portions and
the operation unit includes an engaging portion capable of engaging with the pin.

12. The inhaler according to claim 11, wherein
the cartridge portion includes an opening in a circular shape provided in a center thereof and a plurality of gear grooves at intervals of the same angle as the angle between the housing portions on an inner circumferential surface of the opening,
the operation unit includes a gear fixed to the outer body and having a plurality of gear portions engaged with the gear grooves, and
when the base portion rotates, the cartridge portion rotates, and the gear portion climbs over the gear groove, the engaging portion and the pin are disengaged.

13. The inhaler according to claim 12, wherein
the housing portion includes a drug pocket that houses the drug and a laminate film that seals the drug pocket and
the inner nozzle is formed so as to be able to abut on and break the laminate film with movement of the outer nozzle with respect to the cartridge portion.

14. The inhaler according to claim 13, wherein the cartridge portion includes a guide rail that passes through the plurality of housing portions and in which a tip of the inner nozzle is arranged.

15. The inhaler according to claim 14, wherein
the operation unit moves the outer nozzle in a direction approaching the cartridge portion and
the outer nozzle includes between the outer nozzle and the cartridge portion the elastic portion that energizes between the outer nozzle and the cartridge portion.

16. The inhaler according to claim 15, wherein
the outer nozzle forms a channel along the axial center thereof and includes a slit along the axial center thereof at one end and
the inner nozzle is formed in a cylindrical shape and includes a plate portion provided in a portion of an outer circumferential surface thereof and guided along the slit when the inner nozzle moves inside the outer nozzle and a tube portion provided in a portion of the outer circumferential surface thereof and forming the channel in a direction perpendicular to the axial center of the inner nozzle.

17. The inhaler according to claim 1, further comprising:
a cover provided in the outer body rotatably with respect to the mouth portion and having an engagement protrusion formed on one end side in a rotation direction thereof; and
an operation unit including an operation body operable in the rotation direction of the cover and an engaged portion capable of engaging with the engaging portion provided by being arranged on one end of the operation body on a side of the opening of the engagement protrusion.

18. The inhaler according to claim 17, wherein
an end of the nozzle portion is provided by being separated from the mouth portion and
the operation unit includes a valve body in a plate shape arranged between the nozzle portion and the mouth portion and which recedes from between the nozzle portion and mouth portion with rotation of the operation body.

19. The inhaler according to claim 17, wherein
the cartridge portion is formed in a disc shape and includes a plurality of pins rotatably supported inside the outer body and arranged on an outer circumferential side thereof at intervals of a same angle as the housing portions and
the operation unit includes an engaging portion capable of engaging with the pin.

20. The inhaler according to claim 19, wherein
the nozzle portion includes an outer nozzle formed movably with respect to the cartridge portion, an inner nozzle movable inside the outer nozzle along an axial center of the outer nozzle, and energizing member that energizes the outer nozzle and the inner nozzle in a direction separating from the cartridge portion by energizing the outer nozzle,
the cartridge portion includes a guide rail that passes through the plurality of housing portions and in which a tip of the inner nozzle is arranged, and
the operation unit includes a pressing means that moves the cartridge portion by pressing the outer nozzle.

21. The inhaler according to claim 19, further comprising: an indicator panel formed in a disc shape and having an information indicator on which information about the housing portion is written on a principal surface thereof, a transfer means that transfers rotation of the cartridge portion to the indicator panel, and an indicator having a window portion formed in a portion of an outer surface of the outer body opposite to the information indicator.

22. The inhaler according to claim 1, wherein
the cartridge portion includes a plurality of pins rotatably supported inside the outer body and arranged on an outer circumferential side thereof at intervals of a same angle as the housing portions and also includes
an operation unit including an engaging portion capable of engaging with the pin and an operation body that rotates the engaging portion engaging with the pin along an outer circumference of the cartridge portion.

23. The inhaler according to claim 22, wherein
the cartridge portion has an opening having a plurality of gear grooves formed at intervals of a same angle as the plurality of pins on an inner circumferential surface thereof formed in a center thereof,
the operation unit includes the engaging portion and the operation body and also includes a gear member having a lever member that rotates with respect to the outer body and a plurality of gear portions fixed to the outer body, capable of meshing with the gear grooves, and capable of climbing over the gear grooves, and
the engaging portion engages with the pin until the gear portion climbs over the gear groove.

24. The inhaler according to claim 23, wherein the outer body includes a guide portion in an arc shape formed so as to be able to guide the engaging portion in a circumferential direction of the cartridge portion when the pin and the engaging portion are engaged and also to guide the engaging portion to a center side of the cartridge portion after the gear portion climbs over the gear groove.

25. The inhaler according to claim 24, wherein
the guide portion includes a first guide portion that guides the engaging portion to a position where the engaging portion engages with the pin and a second guide portion that guides the engaging portion to the center side of the cartridge portion and
the engaging portion engages with the first guide portion and includes a guide protrusion provided in a head portion engaging with the pin and the head portion engaging with the second guide portion.

26. The inhaler according to claim 23, wherein
the nozzle portion includes an outer nozzle formed movably with respect to the cartridge portion, an inner nozzle movable inside the outer nozzle along an axial center of the outer nozzle, and energizing member that energizes the outer nozzle and the inner nozzle in a direction separating from the cartridge portion by energizing the outer nozzle,
the cartridge portion includes a guide rail that passes through the plurality of housing portions and in which a tip of the inner nozzle is arranged, and
the operation unit includes a pressing means that moves the cartridge portion by pressing the outer nozzle.

27. The inhaler according to claim 23, further comprising: an indicator panel formed in a disc shape and having an information indicator on which information about the housing portion is written on a principal surface thereof, a transfer means that transfers rotation of the cartridge portion to the indicator panel, and an indicator having a window portion formed in a portion of an outer surface of the outer body opposite to the information indicator.

28. The inhaler according to claim 23, wherein
an end of the nozzle portion is provided by being separated from the mouth portion and
the operation unit includes a valve body in a plate shape arranged between the nozzle portion and the mouth portion and which recedes from between the nozzle portion and mouth portion with rotation of the operation body.
